(19) Europäisches Patentamt
European Patent Office
Office européen des brevets

(11) EP 3 766 499 A1

(12) EUROPEAN PATENT APPLICATION
published in accordance with Art. 153(4) EPC

(43) Date of publication:
20.01.2021 Bulletin 2021/03

(21) Application number: 19766516.9

(22) Date of filing: 13.03.2019

(51) Int Cl.:
A61K 31/65 (2006.01)
A61P 35/00 (2006.01)
A61P 37/04 (2006.01)
A61P 43/00 (2006.01)

(86) International application number:
PCT/JP2019/010236

(87) International publication number:
WO 2019/177011 (19.09.2019 Gazette 2019/38)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 13.03.2018 JP 2018045935

(71) Applicant: Osaka University
Suita-shi, Osaka 565-0871 (JP)

(72) Inventors:
- IWAHORI, Kota
Suita-shi, Osaka 565-0871 (JP)
- WADA, Hisashi
Suita-shi, Osaka 565-0871 (JP)
- NOGUCHI, Yuki
Suita-shi, Osaka 565-0871 (JP)
- KUMANOGOH, Atsushi
Suita-shi, Osaka 565-0871 (JP)

(74) Representative: Arends, William Gerrit
Marks & Clerk LLP
15 Fetter Lane
London EC4A 1BW (GB)

# (54) TUMOR IMMUNOPOTENTIATOR

(57) The present invention provides a less expensive immunotherapy drug for tumor.

Tumor immunity can be activated by at least one member selected from the group consisting of tetracycline-based compounds and pharmaceutically acceptable salts thereof.

Fig.1

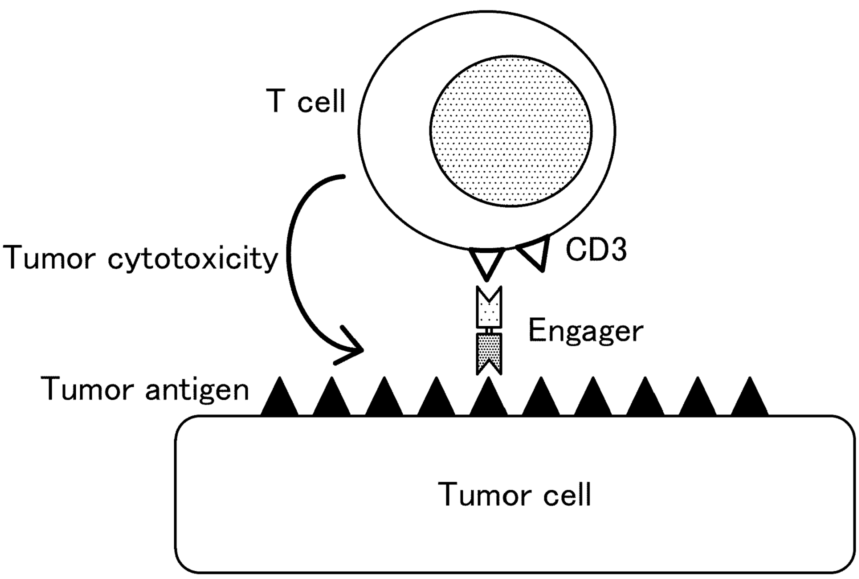




Printed by Jouve, 75001 PARIS (FR)

## Description

### Technical Field

The present specification discloses a tumor immunomodulator.

### Background Art

As cancer immunotherapies, immune checkpoint inhibitor therapies including use of anti-PD-1 antibodies have been employed in clinical trials. Such therapies are effective, and their usefulness is suggested.
Non-patent literature 1 and 2 indicate that administration of a chemically modified form of tetracycline to tumor cells inhibits the growth of the tumor cells to cause cytotoxicity.

### Citation List

### Non-Patent Literature

NPL 1: Anticancer Drugs, 2013 Sep; 24(8)799-809
NPL 2: Curr Med Chem, 2001 Feb; 8(3)271-279

### Summary of Invention

### Technical Problem

However, immune checkpoint inhibitors are effective only for some patients, and biomarkers for predicting patients for whom such a treatment will be effective are still in development. Moreover, immune checkpoint inhibitors have a problem of very high drug prices. Chimeric antigen receptor (CAR) gene-modified T-cell therapy, development of which has been advanced as another cancer immunotherapy, is also a very expensive therapy, and its price is a barrier to practical application. In light of such a situation, the development of an inexpensive immunotherapy that is effective for a larger number of cancer patients is an urgent issue.

### Solution to Problem

In these circumstances, the present inventors found from existing drugs with expired patent terms that a tetracycline-based compound is effective for activating (modulating) tumor immunity.
The inventions described in the present specification have been accomplished based on the above finding, and include the following embodiments.

Item 1. A tumor immunomodulator comprising at least one member selected from the group consisting of a tetracycline-based compound represented by the following formula (I) and its pharmaceutically acceptable salt:

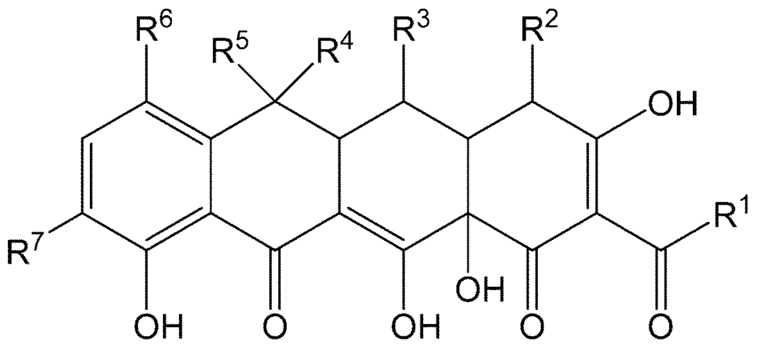

(I)

wherein

$R^1$ is a $C_{1-3}$ lower alkyl group or a group represented by the following formula (II):

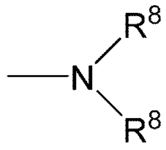 (II)

wherein $R^8$ is a hydrogen atom or a $C_{1-3}$ lower alkyl group; $R^2$ is a group represented by the following formula (III):

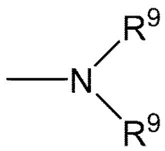 (III)

wherein $R^9$ is a $C_{1-3}$ lower alkyl group or a hydrogen atom; $R^3$ is a hydrogen atom, a hydroxyl group, or a $C_{1-3}$ lower alkyl group;
$R^4$ and $R^5$ are the same or different and each is a hydrogen atom, a hydroxyl group, or a $C_{1-3}$ lower alkyl group, or $R^4$ and $R^5$ taken together are a methylene group;
$R^6$ is a hydrogen atom, halogen, or a group represented by the following formula (IV):

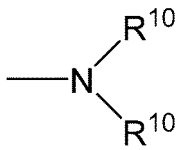 (IV)

wherein $R^{10}$ is a hydrogen atom or a $C_{1-3}$ lower alkyl group; and $R^7$ is a hydrogen atom, a $C_{1-3}$ lower alkyl group, $-NH-CO-CH_2-NH-C\ (CH_3)_3$, or $-CH_2-NH-CH_2-C(CH_3)_3$.

Item 2. The tumor immunomodulator according to Item 1, wherein the $C_{1-3}$ lower alkyl group is a methyl group.

Item 3. The tumor immunomodulator according to Item 1, wherein the compound represented by formula (I) is at least one member selected from the group consisting of demethyl chlortetracycline, meclocycline, tetracycline, chlortetracycline, doxycycline, minocycline, and oxytetracycline.

Item 4. The tumor immunomodulator according to any one of Items 1 to 3, for use in combination with an anti-tumor drug.

Item 5. The tumor immunomodulator according to Item 4, wherein the anti-tumor drug is a tumor immunology agent.

Item 6. The tumor immunomodulator according to Item 5, wherein the tumor immunotherapy agent is at least one member selected from the group consisting of immune checkpoint inhibitors, CAR-T cell drugs, bispecific molecule drugs, and cancer vaccines.

Item 7. A composition for tumor treatment comprising the tumor immunomodulator according to any one of Items 1 to 3 and a tumor immunotherapy agent.

Item 8. The composition for tumor treatment according to Item 7, wherein the tumor immunotherapy agent is at least one member selected from the group consisting of immune checkpoint inhibitors, CAR-T cell drugs, bispecific molecule drugs, and cancer vaccines.

Advantageous Effects of Invention

A tetracycline-based compound can activate tumor immunity.

Brief Description of Drawings

Fig. 1 outlines a system for evaluating the anti-tumor activity of T cells *in vitro.*

Fig. 2 shows an effect of a test drug on the anti-tumor activity of T cells *in vitro.* "BiTE" indicates the addition of BiTE alone, and "BiTE+DMC" indicates the addition of BiTE and demethyl chlortetracycline (DMC) in combination.

Fig. 3 shows an effect of a test drug on the tumor-cytotoxic activity of $CD8^+T$ cells *in vitro.* "BiTE" indicates the addition of BiTE alone, and "BiTE+DMC" indicates the addition of BiTE and demethyl chlortetracycline (DMC) in combination.

Fig. 4 shows an effect of a test drug on the presence ratio of granzyme B-expressing $CD8^+T$ cells *in vitro.* "BiTE" indicates the addition of BiTE alone, and "BiTE+DMC" indicates the addition of BiTE and demethyl chlortetracycline (DMC) in combination.

Fig. 5 shows an effect of a test drug on the proliferative capacity of $CD8^+T$ cells *in vitro.* "BiTE" indicates the addition of BiTE alone, and "BiTE+DMC" indicates the addition of BiTE and demethyl chlortetracycline (DMC) in combination.

Fig. 6 shows an effect of a test drug on the induction of CMV (cytomegalovirus)-specific cytotoxicity T cells *in vitro.* Fig. 6A shows a scattergram of FACS analysis 7 and 14 days after the CMV treatment. The CMV $Tetramer^+$ cells are fractionated in fraction P7. Fig. 6B shows the presence ratio of the CMV $Tetramer^+$ cells 14 days after the CMV treatment.

Fig. 7A shows an effect of a test drug on the cytotoxic activity of T cells in lung cancer tissue *in vitro.* Fig. 7B shows an effect of a test drug on the presence ratio of IFNγ-producing $CD8^+T$ cells *in vitro.* Fig. 7C shows a scattergram of FACS analysis. The IFNy-producing $CD8^+T$ cells are fractionated in fraction P8.

Fig. 8A shows a test drug administration protocol. Fig. 8B shows a change in tumor volume. In Fig. 8B, the dashed line shows a vehicle group and the solid line shows a test drug administration group.

Fig. 9 shows an effect of combined use of demethyl chlortetracycline and an anti-PD-LI antibody. P indicates a significant difference.

Fig. 10 shows that an effect of enhancing an anti-tumor action of an immune checkpoint inhibitor containing demethyl chlortetracycline depends on $CD8^+$ T cells. P indicates a significant difference.

Fig. 11 shows an effect of demethyl chlortetracycline on the presence ratio of cancer antigen-specific $CD8^+T$ cells in peripheral mouse blood. P indicates a significant difference.

## Description of Embodiments

### 1. Tumor Immunomodulator

In the present specification, the tumor immunomodulator comprises, as an active ingredient, a tetracycline-based compound or its pharmaceutically acceptable salt.

The type of the tumor is not particularly limited, and the tumor may be a benign tumor or a malignant tumor, and is preferably a malignant tumor. The tumor also includes an epithelial tumor and a non-epithelial tumor, and is preferably an epithelial tumor. In the present invention, the tumor is most preferably a malignant epithelial tumor.

Examples of malignant tumors include malignant respiratory-system tumors, which develop in the trachea, bronchus, lung, etc.; head and neck tumors; malignant gastrointestinal tumors, which develop in the esophagus, stomach, duodenum, jejunum, ileum, cecum, appendix, ascending colon, transverse colon, sigmoid colon, rectum, anal region, etc.; liver cancer; pancreatic cancer; malignant urological tumors, which develop in the bladder, ureter, or kidney; malignant female-reproductive-system tumors, which develop in the ovary, fallopian tube, uterus, etc.; breast cancer; prostate cancer; skin cancer; malignant endocrine-system tumors, such as the hypothalamus, pituitary gland, thyroid gland, parathyroid gland, and adrenal gland; malignant central-nervous-system tumors; solid tumors, such as malignant tumors that develop in the bone and soft tissue; malignant hematopoietic tumors, such as of myelodysplastic syndrome, acute lymphocytic leukemia, acute myeloid leukemia, chronic lymphocytic leukemia, chronic myeloid leukemia, acute myelomonocytic leukemia, chronic myelomonocytic leukemia, acute monocytic leukemia, chronic monocytic leukemia, acute promyelocytic leukemia, acute megakaryocytic leukemia, erythroleukemia, eosinophilic leukemia, chronic eosinophilic leukemia, chronic neutrophilic leukemia, adult T-cell leukemia, hairy-cell leukemia, plasma cell leukemia, multiple myeloma, and malignant lymphoma; and hematopoietic organ tumors, such as malignant lymphatic-system tumors. More preferable examples of malignant tumors include malignant respiratory-system epithelial tumors, and malignant pleural mesothelioma, such as lung cancer (squamous-cell cancer, small-cell cancer, large-cell cancer, and adenocarcinoma); head and neck tumors; malignant gastrointestinal epithelial tumors, such as esophageal cancer, stomach cancer, colorectal cancer (e.g., sigmoid colon cancer and rectal cancer); liver cancer; pancreatic cancer; renal-cell cancer; bladder cancer; malignant melanoma; classical Hodgkin lymphoma; ovarian cancer; breast cancer; and prostate cancer. Most preferably, the malignant tumor is lung cancer.

Tumor immunity is considered to be an organism’s response against tumors based on the cytotoxic activity of T cells against tumor cells. Accordingly, it can be said that activating tumor immunity also means increasing the cytotoxic activity of T cells against tumor cells.

The tetracycline compound used in the present invention is not intended to exert a proliferation inhibition action or a cytotoxic action directly on tumor cells as described in Non-patent Literature 1 and 2. As described in the Examples below, the tetracycline compound has an action of enhancing the tumor-cytotoxic activity of T cells.

Examples of the tetracycline-based compound include those represented by the following formula (I):

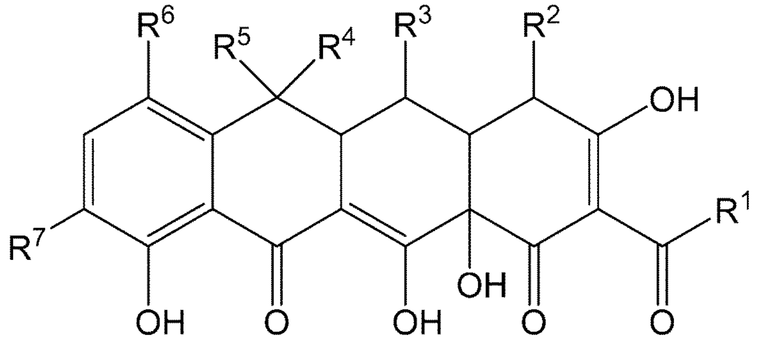

(I)

wherein

$R^1$ is a $C_{1-3}$ lower alkyl group or a group represented by the following formula (II):

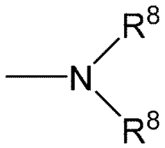

(II)

wherein $R^8$ is a hydrogen atom or a $C_{1-3}$ lower alkyl group; $R^2$ is a group represented by the following formula (III):

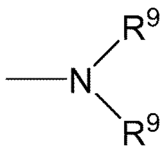

(III)

wherein $R^9$ is a $C_{1-3}$ lower alkyl group or a hydrogen atom; $R^3$ is a hydrogen atom, a hydroxyl group, or a $C_{1-3}$ lower alkyl group;

$R^4$ and $R^5$ are the same or different and each is a hydrogen atom, a hydroxyl group, or a $C_{1-3}$ lower alkyl group, or $R^4$ and $R^5$ taken together are a methylene group;

$R^6$ is a hydrogen atom, halogen, or a group represented by the following formula (IV):

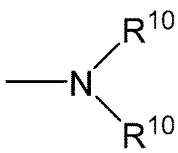

(IV)

wherein $R^{10}$ is a hydrogen atom, or a $C_{1-3}$ lower alkyl group; and $R^7$ is a hydrogen atom, a $C_{1-3}$ lower alkyl group, $-NH-CO-CH_2-NH-C\ (CH_3)_3$, or $-CH_2-NH-CH_2-C(CH_3)_3$.

Examples of $C_{1-3}$ lower alkyl groups include methyl, ethyl, propyl, and isopropyl. The $C_{1-3}$ lower alkyl group is preferably methyl or ethyl, and more preferably methyl.

Halogen is not particularly limited. Examples include chlorine, fluorine, bromine, and iodine. Preferable examples include chlorine.

The tetracycline-based compound is preferably at least one member selected from the group consisting of tetracycline compounds listed in Table 1 below.

Table 1

| | $R^1$ | $R^2$ | $R^3$ | $R^4$ | $R^5$ | $R^6$ | $R^7$ |
|---|---|---|---|---|---|---|---|
| Oxytetracycline | $NH_2$ | $N(CH_3)_2$ | OH | $CH_3$ | OH | H | H |
| Tetracycline | $NH_2$ | $N(CH_3)_2$ | H | $CH_3$ | OH | H | H |
| Demethyl chlortetracycline | $NH_2$ | $N(CH_3)_2$ | H | H | OH | Cl | H |
| Doxycycline | $NH_2$ | $N(CH_3)_2$ | OH | $CH_3$ | H | H | H |
| Minocycline | $NH_2$ | $N(CH_3)_2$ | H | H | H | $N(CH_3)_2$ | H |
| Meclocycline | $NH_2$ | $N(CH_3)_2$ | OH | $CH_2$ | | Cl | H |
| Chlortetracycline | $NH_2$ | $N(CH_3)_2$ | H | $CH_3$ | OH | Cl | H |
| C6-demethyl-C7-chlortetracycline | $NH_2$ | $N(CH_3)_2$ | H | H | OH | Cl | H |
| 2-Acetyl-2-decarboxy-amide-oxytetracycline | $CH_3$ | $N(CH_3)_2$ | OH | $CH_3$ | OH | H | H |
| Chelocardin | $CH_3$ | $NH_2$ | H | $CH_3$ | H | H | $CH_3$ |
| Tigecycline | $NH_2$ | $N(CH_3)_2$ | H | H | H | $N(CH_3)_2$ | $NH-CO-CH_2-NH-C(CH_3)_3$ |
| Omadacycline | $NH_2$ | $N(CH_3)_2$ | H | H | H | $N(CH_3)_2$ | $CH_2-NH-CH_2-C(CH_3)_3$ |

The tetracycline-based compound is more preferably at least one member selected from the group consisting of demethyl chlortetracycline, meclocycline, tetracycline, chlortetracycline, doxycycline, minocycline, and oxytetracycline.

The tetracycline-based compounds and salts thereof disclosed in the present specification are known. Accordingly, the methods for producing tetracycline-based compounds and salts thereof are also known.

Salts of tetracycline-based compounds are not limited as long as they are pharmaceutically acceptable salts. For example, salts of a tetracycline-based compound containing an amine or other basic group can be prepared by reacting a tetracycline-based compound with a preferable organic or inorganic acid to generate an anionic salt. Examples of anionic salts include acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylsulfate, mucate, napsylate, nitrate, pamoate, pantothenate, phosphate/diphosphate, polygalacturonate, salicylate, stearate, subacetate, succinate, sulfate, tannate, tartrate, teoclate, tosylate, and triethiodide salts, and the like. The salt is preferably hydrochloride or dihydrochloride.

The tetracycline-based compound can generate a cationic salt by a reaction with a suitable base. Cationic salts may be prepared with a base that gives pharmaceutically acceptable ions. Examples include alkali metal salts (particularly sodium and potassium), alkaline earth metal salts (particularly calcium and magnesium), aluminum salts, ammonium salts; and salts of basic amino acids such as trimethyl amine, triethyl amine, morpholine, pyridine, piperidine, picoline, dicyclohexylamine, N,N'-dibenzylethylenedimine, 2-hydroxyethyl amine, bis-(2-hydroxyethyl)amine, tri-(2-hydroxyethyl)amine, procaine, dibenzylpiperidine, dehydroabietylamine, N,N'-bis dehydroabietylamine, glucamine, N-methylglucamine, collidine, quinine, quinoline, lysine, and arginine.

The tumor immunomodulator includes a tetracycline-based compound or its pharmaceutically acceptable salt. The tumor immunomodulator can be prepared by combining a tetracycline-based compound or its pharmaceutically acceptable salt and a suitable carrier or additive. Examples of the carrier or additive for use in the preparation of the tumor immunomodulator include those commonly and conventionally used for drugs according to the dosage form of the tumor immunomodulator, such as excipients, binders, disintegrates, lubricants, colorants, sweetening agents, flavoring agents, surfactants, and the like.

The dosage form of the tumor immunomodulator when orally administered is not particularly limited. Examples include tablets, powders, granules, capsules (including hard capsules and soft capsules), liquids, pills, suspensions, emulsions, and the like. Examples of a tumor immunomodulator that is not orally administered include injections, drops, suppositories, nasal drops, pulmonary administration agents, and the like.

When the tumor immunomodulator is prepared as an oral solid composition, such as tablets, powders, granules, pills, or capsules, examples of carriers include excipients, such as lactose, sucrose, sodium chloride, glucose, urea, starch, calcium carbonate, kaolin, crystalline cellulose, silicic acid, methylcellulose, glycerol, sodium alginate, and gum arabic; binders, such as simple syrup, glucose solutions, starch solutions, gelatin solutions, polyvinyl alcohol, polyvinyl ether, polyvinylpyrrolidone, carboxymethylcellulose, shellac, methylcellulose, ethylcellulose, water, ethanol, and potassium phosphate; disintegrates, such as dry starch, sodium alginate, agar powder, laminaran powder, sodium hydrogen carbonate, calcium carbonate, polyoxyethylene sorbitan fatty acid esters, sodium lauryl sulfate, stearic acid monoglyceride, starch, and lactose; disintegrating inhibitors, such as sucrose, stearic acid, cacao butter, and hydrogenated oils; absorption enhancers, such as sodium lauryl sulfate; wetting agents, such as glycerol and starch; adsorbents, such as starch, lactose, kaolin, bentonite, and colloidal silica; lubricants, such as purified talc, stearates, boric acid powder, and polyethylene glycol. Further, such tablets may be coated with typical coating materials as required, to prepare, for example, sugar-coated tablets, gelatin-coated tablets, enteric-coated tablets, film-coated tablets, double-layered tablets, or multi-layered tablets.

When the tumor immunomodulator is prepared as an oral solid composition in the form of a pill, examples of carriers include excipients, such as glucose, lactose, starch, cacao butter, hydrogenated vegetable oil, kaolin, and talc; binders, such as gum arabic powder, tragacanth powder, and gelatin; disintegrators, such as laminaran and agar; and the like.

When the tumor immunomodulator is prepared as an oral solid composition in the form of a capsule, a capsule is prepared by mixing an active ingredient with various carriers listed above and filling the mixture into a hard capsule or a soft capsule.

When the preparation is a liquid, it may be an aqueous or oily suspension, solution, syrup, or elixir, and the preparation is prepared according to a conventional method using a common additive.

When the tumor immunomodulator is prepared as an injection, examples of carriers include diluents, such as water, ethyl alcohol, macrogol, propylene glycol, ethoxylated isostearyl alcohol, polyoxylated isostearyl alcohol, and polyoxyethylene sorbitan fatty acid esters; pH-adjusting agents, such as sodium citrate, sodium acetate, and sodium phosphate; buffers, such as dipotassium phosphate, trisodium phosphate, sodium hydrogen phosphate, and sodium citrate; stabilizers, such as sodium pyrosulfite, EDTA, thioglycolic acid, and thiolactic acid; and molding agents for use in freeze-drying the tumor immunomodulator, such as sugar such as mannitol, inositol, maltose, sucrose, lactose, and like. In this case, glucose or glycerol may be incorporated in the preparation in an amount sufficient to prepare an isotonic solution. General auxiliary solvents, soothing agents, topical anesthetics, and the like may also be added. Subcutaneous, intramuscular, and intravenous injections can be prepared according to common methods by adding the carriers mentioned above.

When the preparation is in the form of drops, it is prepared by dissolving an administration compound in a base isotonic electrolyte infusion preparation, such as saline and Ringer's solution.

The tumor immunomodulator in the present invention may be an oral composition or a parenteral composition, but is preferably an oral composition.

One or more tetracycline-based compounds or pharmaceutically acceptable salts thereof may be contained in the tumor immunomodulator. Specifically, the tumor immunomodulator may contain at least one member selected from the group consisting of tetracycline-based compounds and their pharmaceutically acceptable salts.

The dose of the tetracycline-based compound or its pharmaceutically acceptable salt is known. Accordingly, the dose of the tumor immunomodulator can also be determined in terms of the dose of the known tetracycline-based compound. For example, the dose of the tetracycline-based compound or its pharmaceutically acceptable salt in the case of oral administration is about 0.1 mg to 1,000 mg/kg (adult), or 0.02 mg to 100 mg/kg (children) per day. In the case of an injection or intravenous infusion, the dose is about 0.1 mg to 500 mg/kg (adult), or 0.02 mg to 50 mg/kg (children) per day. The dose can be suitably adjusted based on age, symptoms, tumor size, and administration conditions of other drugs. When two or more tetracycline-based compounds or their pharmaceutically acceptable salts are administered in combination, the total content of the tetracycline-based compounds or their pharmaceutically acceptable salts is preferably in the above dose range.

The tetracycline-based compound or its pharmaceutically acceptable salt may be used in combination with other drugs. Other drugs that are used in combination with the tetracycline-based compound or its pharmaceutically acceptable salt are not particularly limited, but an anti-tumor drug is preferred.

Usable anti-tumor drugs include those that are generally used together with anticancer drugs. The anti-tumor drug can be suitably selected from alkylating agents, antimetabolites, anti-tumor antibiotics, microvascular inhibitors,

hormones or hormone-like drugs, platinum drugs, topoisomerase inhibitors, cytokines, antibody drugs, tumor immunotherapy agents (radioimmunotherapeutic agents, non-specific immune stimulators, immune checkpoint inhibitors, CAR-T cell drugs, BiTE drugs, cancer vaccines, etc.), molecularly targeted drugs, and other anti-tumor drugs according to the target tumor.

Although there is no limitation, examples of alkylating agents include cyclophosphamide, ifosfamide, busulfan, melphalan, bendamustine hydrochloride, nimustine hydrochloride, ranimustine, dagarbazine, and procarbazine hydrochloride temozolomide; examples of antimetabolic agents include methotrexate, pemetrexed sodium, fluorouracil, doxyfluridine, capecitabine, tegafur, cytarabine, cytarabine octophosphate hydrate, enocitabine, gemcitabine hydrochloride, mercaptopurine hydrate, fludarabine phosphate, nelarabine, pentostatin, cladribine, calcium levofolinate, calcium phorinate, hydroxycarbamide, L-asparaginase, and azacitidine; examples of anti-tumor antibiotics include doxorubicin hydrochloride, daunorubicin hydrochloride, pyrarubicin, epirubicin hydrochloride, idarubicin hydrochloride, acrarubicin hydrochloride, amrubicin hydrochloride, mitoxantrone hydrochloride, mitomycin C, actinomycin D, bleomycin, pepromycin sulfate, and dinostatin styramer; examples of microvascular inhibitors include vincristine sulfate, vinblastine sulfate, vindesine sulfate, vinorelbine tartrate, paclitaxel, docetaxel hydrate, and eribulin mesylate; examples of hormones or hormone-like drugs (hormone agents) include anastrozole, exemestane, letrozole, tamoxifen citrate, tremifen citrate, fulvestrant, flutamide, bicalutamide, medroxyprogesterone acetate, estramustine phosphate sodium hydrate, goserelin acetate, and leuprorelin acetate; examples of platinum drugs include cisplatin, myriplatin hydrate, carboplatin, nedaplatin, and oxaliplatin; examples of topoisomerase inhibitors include topoisomerase I inhibitors, such as irinotecan hydrochloride hydrate and nogitecan hydrochloride, and topoisomerase II inhibitors, such as etoposide and sobuzoxane; examples of cytokines include interferon gamma-la, teceleukin, and celmoleukin; examples of antibody agents include trastuzumab, rituximab, gemtuzumab ozogamicin, bevacizumab, cetuximab, panitumumab, and alemtuzumab; examples of radioimmunotherapy agents include ibritumomab, and tiuxetan combination drugs; examples of molecular targeted drugs include gefitinib, imatinib mesylate, bortezomib, erlotinib hydrochloride, sorafenib tosilate, sunitinib malate, thalidomide, nilotinib hydrochloride hydrate, dasatinib hydrate, lapatinib tosilate hydrate, everolimus, lenalidomide hydrate, dexamethasone, temsirolimus, vorinostat, tretinoin, and tamibarotene; examples of non-specific immune active agents include OK-432, dried BCG, trametas versicolor polysaccharide preparation, lentinan, and ubenimex; examples of immune checkpoint inhibitors include atezorizumabu, nivolumab, pembrolizumab, ipilimumab, durvalumab, avelumab, and tremelimumab; examples of CAR-T cell drugs include tisagenlecleucel; examples of bispecific molecule drugs include BiTE (trademark) drugs; examples of cancer vaccines include sipuleucel-T; and others, such as aceglatone, porfimer sodium, talaporfin sodium, ethanol, and trioxide arsenic. Herein, the bispecific molecule means a molecule that contains an antigen-binding domain that binds to at least one type of surface antigen of a T cell and an antigen-binding domain that binds to at least one type of surface antigen of a tumor cell. The number of antigen-binding domains that bind to at least one type of surface antigen may be one, two, or more per antigen. For example, the antigen-binding domain that binds to at least one type of surface antigen may be a combination of Fab fragments of the same type, a combination of variable regions of the same type, a combination of sc-Fv of the same type, a combination of a heavy-chain Fab fragment and a light-chain Fab fragment derived from one antibody, a combination of a heavy-chain variable region and a light-chain variable region derived from one antibody, or a combination of a heavy chain and sc-Fv derived from one antibody. The surface antigens of a T cell are present on the surface of the T cell; the surface antigens can be any surface antigen as long as at least one antigen-binding domain present in a bispecific molecule or a trispecific molecule can bind to one of the surface antigens. The surface antigens of a T cell preferably include the surface antigens of a cytotoxic T cell. Examples of surface antigens of a T cell include CD3, CD8, TCR, CTLA-4, PD1, Tim3, CD27, CD28, CD40, CD134 (OX40), CD137 (4-1BB), and CD278 (ICOS), with CD3 being preferable. The surface antigens of a tumor cell are present on the surface of the tumor cell; the surface antigens can be any surface antigen as long as at least one antigen-binding domain present in a bispecific molecule can bind to one of the surface antigens. The surface antigens of a tumor cell include EphA1 (ephrin type-A receptor 1), EphA2, $FolR^1$ (folate receptor 1), EpCAM (epithelial cell adhesion molecule), CD19, Her1 (v-erb-b2 erythroblastic leukemia viral oncogene homolog 1: ErbB1), Her2, CD20, EGFR (epidermal growth factor receptor), $CCR^4$ (C-C chemokine receptor type 4), CEA (carcinoembryonic antigen), GD2, GD3, CD22, CD30, CD33, CD70, CD123, EGFRvIII, MUC1 (Mucin1), PSCA (prostate stem cell antigen), PSMA (prostate-specific membrane antigen), HLA-A1+NY-ESO1 (HLA-A1 restricted NY-ESO1), HLA-A2+NY-ESO1 (HLA-A2 restricted NY-ESO1), and HLA-A3+NY-ESO1 (HLA-A3 restricted NY-ESO1). The bispecific molecule includes BiTE (trade name) and a bispecific antibody. Examples of BiTE (trade name) include a bispecific molecule that targets CD19 and CD3, and a bispecific molecule that targets EphA2 and CD3. Examples of the bispecific antibody also include an antibody that targets EpCAM and CD3.

From the viewpoint of effects of actions of a tetracycline-based compound, a tumor immunotherapy agent is preferred as an anti-tumor drug, and at least one member selected from the group consisting of immune checkpoint inhibitors, CAR-T cell drugs, bispecific molecule drugs, and cancer vaccines is more preferred. Of the tumor immunotherapy agents, an immune checkpoint inhibitor (atezorizumabu, nivolumab, pembrolizumab, ipilimumab, durvalumab, avelumab, tremelimumab, etc.) is most preferred.

The anti-tumor drug can be administered according to a known method. In determining the dose of the anti-tumor drug, according to the measurement method of tumor-cytotoxic activity using peripheral blood mononuclear cells described in Example 1 below, the intensity of tumor-cytotoxic activity of T cells in peripheral blood mononuclear cells may be evaluated for each patient, and the dose may be determined according to the intensity.

Embodiments of the combination use (combination) of the tetracycline-based compound or its pharmaceutically acceptable salt according to the present invention and an anti-tumor drug are not particularly limited as long as the effects of the present invention are attained. For example, the tetracycline-based compound or its pharmaceutically acceptable salt may be concurrently administered with an anti-tumor drug; the tetracycline-based compound or its pharmaceutically acceptable salt may be administered before or after the administration of an anti-tumor drug. In this case, the tetracycline-based compound or its pharmaceutically acceptable salt and an anti-tumor drug may be administered alternately. Furthermore, it is also possible that an anti-tumor drug is first administered, and then the administration of the tetracycline-based compound or its pharmaceutically acceptable salt may be started in accordance with the degree of reduction in tumor tissue from the middle of the administration of the anti-tumor drug. Conversely, it is also possible that the tetracycline-based compound or its pharmaceutically acceptable drug is first administered, and then the administration of an anti-tumor drug is started from the middle of the administration of the tetracycline-based compound or its pharmaceutically acceptable drug.

As long as the tetracycline-based compound or its pharmaceutically acceptable salt according to the present invention is used in combination with an anti-tumor drug, it may be administered as a single dose, a continuous dose, or an intermittent dose. For example, if the tetracycline-based compound or its pharmaceutically acceptable salt according to the present invention is administered before the administration of the anti-tumor drug, it may be administered 7 days or 3 days before the start of the administration of the anti-tumor drug once a day daily for about 14 to 21 days. Alternatively, the tetracycline-based compound or its pharmaceutically acceptable salt may be administered daily during the administration course of the anti-tumor drug.

The tetracycline-based compound or its pharmaceutically acceptable salt according to the present invention is prepared as a pharmaceutical composition in various forms (dosage forms), depending on its administration route (administration method), and is administered to a target tumor patient in combination with an anti-tumor drug.

Patients eligible for the tumor immunomodulator are not limited as long as the patients are eligible for tumor immunotherapy. Examples of patients include those who have one of the tumors listed above, and have not undergone a treatment, or have already undergone some tumor treatment, patients in tumor treatment, or patients whose tumors have recurred or metastasized. Preferable examples of the tumor treatment include surgical tumor resection, chemotherapy, radiation therapy, and the like.

## 2. Composition for Tumor Treatment

The composition for tumor treatment includes the tumor immunomodulator described in section 1 above, and an anti-tumor drug. The anti-tumor drug is preferably a tumor immunotherapy agent. The tumor immunotherapy agent is more preferably at least one member selected from the group consisting of immune checkpoint inhibitors, CAR-T cell drugs, bispecific molecule drugs, and cancer vaccines. For the dose, the explanation in section 1 above is incorporated here.

The composition for tumor treatment containing a tumor immunomodulator and an anti-tumor drug includes the following embodiments:

(i) the tumor immunomodulator and the anti-tumor drug are contained in the form of a mixture in the same preparation (combination drug),
(ii) the tumor immunomodulator alone or a preparation containing the tumor immunomodulator, and the anti-tumor drug alone or a preparation containing the anti-tumor drug are individually packaged as separate preparations and sold as a combined (kit),
(iii) the tumor immunomodulator alone or a preparation containing the tumor immunomodulator, and the anti-tumor drug alone or a preparation containing the anti-tumor drug are separate preparations, and these are sold in combination as a package, or
(iv) the tumor immunomodulator alone or a preparation containing the tumor immunomodulator, and the anti-tumor drug alone or a preparation containing the anti-tumor drug are individually packaged as separate preparations, marketed through separate distribution channels, and used in combination at use.

## 3. Tumor Treatment Method

The present disclosure includes a method for treating tumors using the tumor immunomodulator and the composition for tumor treatment. For the methods and targets of administration of the tumor immunomodulator and the

composition for tumor treatment, the explanation described in section 1 above is incorporated here.

Examples

The present invention is described in detail with reference to the Examples below. However, the present invention is not limited to the Examples in interpretation of the invention.

I. Experimental Protocol

1. Measurement of Tumor-Cytotoxic Activity Using Peripheral Blood Mononuclear Cells and Evaluation of Each Test Drug

As shown in Fig. 1, *in vitro,* a PBMC (peripheral blood mononuclear cells : PBMC) group was contacted with tumor cells via an engager such as BiTE (registered trademark), and cultured. Thereafter, by measuring the degree of cytotoxicity to the tumor cells (tumor-cytotoxic activity) by the PBMCs, the anti-tumor activity of T cells in the PBMCs was evaluated.

(1) A U251 cell line was suspended in a RPMI1640 medium containing 10% FBS (a 10% FBS-supplemented RPMI1640 medium), and then detached with trypsin. The cell line was suspended in a 10% FBS-supplemented RPMI1640 medium such that the cell count was $1\times10^5$/mL, thereby preparing a cell suspension. The cell suspension was seeded in a 96-well plate at a rate of 100 μL/well, and incubated in a wet incubator at 37°C for 18 hours in the presence of 5% $CO_2$ gas.
(2) Peripheral blood (heparin blood collection) was collected from healthy people, and peripheral blood mononuclear cells (PBMCs) were collected using Lymphoprep™ (Alere Technologies AS) in accordance with the included protocol. Specifically, the collected peripheral blood was slowly layered over Lymphoprep™ contained in the tube, followed by centrifugation at 400 g for 50 minutes, thereby collecting a lymphocyte layer. After a 10% FBS-supplemented RPMI 1640 medium was added to the collected solution, and centrifugation was performed, the supernatant was removed. The pellet of the cells was suspended in a 10% FBS-supplemented RPMI 1640 medium, and the cell count was adjusted to 1 x $10^6$/mL. The PBMCs were collected immediately before step (3) described below.
(3) EphA 2-CD3 BiTE (registered trademark) containing an antigen-binding domain for EphA2 and an antigen-binding domain for CD3 in the molecule was prepared at 400 ng/mL in a 10% FBS-supplemented RPMI 1640 medium ("BiTE solution"). Each test drug was adjusted to 10 μM in the BiTE solution. 50 μL of the suspension of PBMCs and 50 μL of the test drug-containing BiTE solution were added to each well of the 96-well plate subjected to incubation in step (1) so that the final concentration of BiTE was 100 ng/mL and the final concentration of each test drug was 2.5 μM. For a control, wells each containing 50 μL of the suspension of PBMCs and 50 μL of a Bite solution free of a test drug, and wells each containing 50 μL of the suspension of PBMCs and 50 μL of a 10% FBS-supplemented RPMI 1640 medium were prepared in the 96-well plate subjected to incubation in step (1). The amount of the 10% FBS-supplemented RPMI medium per well was determined so that the total final amount was 200 μL. The 96-well plate obtained after PBMCs and EphA2-CD3 BiTE (registered trademark) were added, or PBMCs, EphA2-CD3 BiTE (registered trademark), and a test drug were added, was subjected to incubation at 37°C for 48 hours.
(4) After the completion of incubation, the medium of each well was collected together with the PBMCs. Further, 200 μL of a 10% FBS-supplemented RPMI medium was added to each well for washing, and the medium used for washing was collected. This operation was repeated three times to wash the wells three times. In this step, the medium first collected and the media collected in the washing were put together into one tube for each well. After washing the wells, 100 μL of a 10% FBS-supplemented RPMI medium and 20 μL of CellTiter 96 (registered trademark) AQueous One Solution Reagent (MTS reagent: Promega Corporation) were added to each well in which the media and the PBMCs were removed. Thereafter, the 96-well microplate was subjected to incubation in a wet incubator at 37°C for about 30 minutes to 1 hour in the presence of 5% carbon dioxide.
(5) The absorbance at 492 nm of each well of the 96-well microplate after the completion of incubation in step (4) was measured with a microplate reader. Subsequently, tumor-cytotoxic activity was calculated in accordance with the following equation.

Absorbance of a well consisting of U251 cell line and PBMCs = A Absorbance of a well containing U251 cell line, PBMCs, and EphA2-CD3 BiTE (registered trademark), or a well containing U251 cell line, PBMCs, EphA2-CD3 BiTE (registered trademark), and a test drug = B

$$\text{Tumor-Cytotoxic Activity (\%)} = [(A-B)/A] \times 100$$

2. Granzyme B Staining

The medium collected in section 1(4) above was centrifuged at 400 g for 5 minutes, and the supernatant was removed to leave the PBMC pellet. Each antibody (CD3, CD4, CD8, and CD45RA) was diluted 100-fold with 2%FBS- and 10mM HEPES-supplemented HBSS medium ("HBSS+2%FBS+10mM HEPES" medium). Such antibody diluents were individually added at 30 μL/tube to the pellet in which the supernatant was removed, followed by incubation at 4°C for 30 minutes. After an HBSS+2%FBS+10mM HEPES medium was added at 200 μL/tube, and centrifugation was performed at 400 g for 5 minutes, the supernatant was removed to leave the pellet. After the remaining PBMCs were suspended by adding a fix buffer at 100 μL/tube, incubation was performed for 30 minutes at 4°C.

The tube obtained after the completion of incubation was centrifuged at 400 g for 5 minutes to remove the supernatant and leave the PBMC pellet. After a wash buffer was added at 200 μL/tube to suspend the pellet, centrifugation was performed at 400 g for 5 minutes to remove the supernatant and leave the PBMC pellet. An anti-Granzyme B antibody was diluted 100-fold in a wash buffer, and the remaining PBMCs were suspended in the antibody diluent (30 μL/tube), followed by incubation for 30 minutes at 4°C. By adding a wash buffer in an amount of 200 μL/tube, centrifugation was performed at 400 g for 5 minutes to remove the supernatant and leave the PBMC pellet. The remaining PBMCs were suspended in a HBSS+2% FBS+10 mM HEPES medium, and the suspension was subjected to FACS analysis.

3. CytoTell Assay

PBMCs were collected from peripheral blood of healthy people according to the method described in section 1(1) above. A solution in which CytoTell red was mixed with the HBSS+2%FBS+10mM HEPES medium was added to the collected PBMC pellet, followed by incubation at 37°C for 30 minutes. After the completion of incubation, the supernatant was removed by centrifugation at 400 g for 5 minutes to leave the PBMC pellet. By adding a 10% FBS-supplemented RPMI medium, the remaining PBMCs were suspended to prepare a suspension of PBMCs having a cell count of 1 x $10^6$/mL.

As in section 1(3) above, a U251 cell line, PBMCs, and BiTE, or a U251 cell line, PBMC, BiTE, and a test drug, were contacted and cultured at 37°C for 96 hours.

After the completion of culture, the culture supernatant was collected, followed by washing in a 10% FBS-supplemented RPMI 1640 medium three times, and the media were also collected. In this step, the medium first collected and the media collected in the washing were put together in one tube for each well.

The collected medium was centrifuged at 400 g for 5 min, and the supernatant was removed to leave the PBMC pellet. Each antibody (CD3, CD4, CD8, and CD45RA) was diluted 100-fold with a HBSS+2%FBS+10mM HEPES medium. The antibody diluents were individually added at 30 μL/tube to the pellet to suspend the remaining PBMCs, followed by incubation at 4°C for 30 minutes.

The tube obtained after the completion of incubation was centrifuged at 400 g for 5 minutes by adding a HBSS+2%FBS+10mM HEPES medium at 200 μL/tube to remove the supernatant and leave the PBMC pellet. The PBMCs left in the HBSS+2%FBS+10mM HEPES medium were suspended, and the suspension was subjected to FACS analysis.

4.CMV-specific CTL Induction (Mixed CMV Peptide/Lymphocytes Culture)

CMV peptides adjusted to 10 μg/mL in a 10% FBS-supplemented RPMI medium was added to PBMCs of healthy people with an HLA type of 24:02, followed by culture in a 96-well plate for 3 days. The medium was replaced with a 10% FBS-supplemented RPMI medium containing 10 μg/mL CMV peptide + 20U/mL IL-2 ± 2.5 μM demethyl chlortetracycline (DMC), and culture was performed for another 4 days (culture for 7 days). The medium containing PBMCs in each well was transferred into a 24-well plate for scale-up, followed by culture for another 3 days in a 10% FBS-supplemented RPMI medium containing 10 μg/mL peptide+20U/mL IL-2±2.5 μM DMC. The medium was replaced with a 10% FBS-supplemented RPMI medium containing 10 μg/mL peptide + 20U/mL IL-212.5 μM DMC, followed by culture for another 4 days (culture for 14 days).

After the completion of culture, centrifugation was performed at 400 g for 5 minutes to remove the supernatant and leave the PBMC pellet; and each antibody (CD3, CD4, and CD8) was diluted 100-fold, and CMV Tetramer was diluted 10-fold in a HBSS+2%FBS+10mM HEPES medium, and added at 30 μL/tube to a PBMC pellet to suspend the PBMCs, followed by incubation at 4°C for 30 minutes. By adding the HBSS+2%FBS+10mM HEPES medium at 200 μL/tube, centrifugation was performed at 400 g for 5 minutes. The supernatant was removed to leave the PBMC pellet.

The PBMCs left in the HBSS+2%FBS+10mM HEPES medium were suspended, and the suspension was subjected to FACS analysis.

5. Collection of T Cells in Lung Cancer Tissue

Regarding the tumor-cytotoxic activity of T cells in lung cancer tissue, cells were collected from lung cancer tissue according to the following method. The tumor-cytotoxic activity was measured as the activity of the cell population.

The cancer tissue was transferred to a 6-cm dish and minced. The cancer tissue was then placed in a tissue stirring solution (tumor dissociation Kit (Miltenyi Biotec) was added to HBSS+2%FBS+10mM HEPES), followed by stirring with a gentle MACS™ Dissociator (Miltenyi Biotec). Incubation was then performed by rotating the mixture in an incubator maintained at 37°C for 30 minutes. Thereafter, the cell-containing stirring solution was passed through a 70-μm mesh to remove the tissue residue, and the filtrate was centrifuged at 600 x g for 10 minutes to collect the precipitate. BD Pharm lyse (BD Biosciences) was added to the cell-containing precipitate, and the mixture was allowed to stand for 2 minutes. An HBSS buffer was added to the cell-containing lysate after being allowed to stand, and centrifugation was performed at 600 x g for 10 minutes to collect the precipitate again. A 30% Percoll solution was added to the recovered precipitate to perform centrifugation at 12,000 x g for 30 seconds, and the precipitate was collected again. The recollected precipitate was washed with an HBSS buffer, and centrifugation was performed at 12,000 x g for 30 seconds to collect cells in the tissue. The tumor-cytotoxic activity of the collected cells was measured in accordance with the same method as in section I.1(1) to (5) above. Since BiTE that binds to CD3 was used, the tumor-cytotoxic activity of cells collected from the lung cancer tissue was considered to be the tumor-cytotoxic activity of T cells in the lung cancer tissue.

6. IFNγ Production Assay

The IFNγ production capacity of cells in all media that were collected in section 5 in accordance with the method described in section 1(4) above was measured using an IFNγ secretion assay kit (Miltenyi Biotec).

The medium collected in section 1(4) above was centrifuged at 400 g for 5 minutes to remove the supernatant. An IFNγ catch reagent diluted 100-fold in a 10% human serum-supplemented AIM medium was added at 100 μL/tube to the remaining PBMC pellet to suspend the PBMCs, followed by incubation at 4°C for 10 minutes.

A 10% human serum-supplemented AIM medium heated to 37°C was dispensed at 1 mL/tube into the tube subjected to the incubation. While stirring the tube, incubation was performed at 37°C for 45 minutes. After the completion of incubation, the tube was cooled on ice again and centrifuged at 400 x g for 5 minutes to remove the supernatant and leave the PBMC pellet.

The PBMCs left in the HBSS+2%FBS+10mM HEPES medium were suspended, and each sample was divided into two (one for isotype staining). Thereafter, centrifugation was performed at 400 g for 5 minutes again, and the supernatant was removed to leave the pellet.

In an HBSS+2%FBS+10mM HEPES medium, each antibody (CD3, CD4, CD8, and CD45RA) was diluted 100-fold, and an anti i-IFNγ antibody was diluted 10-fold. Each of these antibody diluents was added to the remaining PBMCs at 30 μL/tube to suspend the PBMCs, followed by incubation at 4°C for 30 minutes.

After an HBSS+2%FBS+10mM HEPES medium was added at 200 μL/tube to the tube subjected to incubation, and centrifugation was performed at 400 g for 5 minutes, the supernatant was removed. The PBMCs left in the HBSS+2%FBS+10mM HEPES medium were suspended again, and the suspension was subjected to FACS analysis.

7. Examination of Tumor Cell Proliferation Inhibition Action *in Vivo*

CT26WT cells were thawed five days before inoculation, and cultured in a DMEM+10%FBS+1% penicillin/streptomycin medium. Two days before the inoculation of tumor cells, the CT26WT cells were treated with trypsin, followed by passage culture.

After trimming hair at the right dorsal side of BALB/c mice, the CT26WT cells were intradermally inoculated at 2 x $10^5$ cells/mouse. The weight and tumor size of each mouse were measured 6 days after the inoculation of tumor cells. The mice were divided into a test drug administration group and a vehicle group (n=9 each) so that the groups had a uniform weight and tumor size. In the test drug administration group, a test drug was dissolved in saline to 3.0 mg/mL and intraperitoneally administered at 200 μL/mouse in an amount of 30 mg/kg/day. In the vehicle group, only saline was intraperitoneally administered at 200 μL/mouse. The test drug or saline was administered daily for 10 days (Fig. 8A). The tumor size was measured 10 days, 13 days, and 16 days after the tumor cell administration.

II. Example 1: Examination of Action of Activating T-cell Anti-Tumor Activity of Tetracycline-based Compound

According to the method described in section I.1 above, demethyl chlortetracycline (DMC), meclocycline (MC),

tetracycline (TC), chlortetracycline (CTC), and minocycline (MINO) were used as test drugs to examine the action of activating T-cell anti-tumor activity. The difference between the group containing BiTE alone and the group containing BiTE and a test drug in combination was calculated using t-assay wherein n (independent healthy people) in each group was 3. The results are shown in Fig. 2.

The combination of BiTE with a test drug (all the cases of DMC, MC, TC, CTC, and MINO) enhanced a T-cell anti-tumor activity as compared to BiTE alone (DMC, MC, and CTC: $p<0.01$; TC and MINO: $p<0.05$). This clearly indicated that the tetracycline-based compound had an action of activating T-cell anti-tumor activity.

III. Example 2: Examination of Enhancement Action of Tetracycline-based Compound on Tumor-Cytotoxic Activity of $CD8^+$ T Cells

According to the method described in section I.1 above, using demeclocycline (DMC: final concentration of 2.5 μM) as a test drug, an effect of the tetracycline-based compound on the tumor-cytotoxic activity of $CD8^+$T cells in the PBMCs that were individually collected from three healthy people was examined. The $CD8^+$T cells were subjected to negative selection using a $CD8^+$T cell isolation kit (Miltenyi Biotec). As shown in Fig. 3, as compared to the $CD8^+$T cells containing BiTE alone, the $CD8^+$T cells containing BiTE and a test drug in combination had high tumor-cytotoxic activity ($p=0.05$). This suggested that the tetracycline-based compound had an action of enhancing the tumor-cytotoxic activity of $CD8^+$T cells.

IV. Example 3: Examination of Increase in Presence Ratio of Granzyme B-expressing $CD8^+$T Cells by Tetracycline-based Compound

According to the method described in section 1.2 above, using demethyl chlortetracycline (DMC: final concentration of 2.5 μM) as a test drug, an effect of the tetracycline-based compound on the presence ratio of the granzyme B-expressing $CD8^+$T cells in the PBMCs that were individually collected from four healthy people was examined. As shown in Fig. 4, as compared to the PBMCs containing BiTE alone, the presence ratio of the granzyme B-expressing $CD8^+$T cells was high ($p = 0.01$) in the PBMCs containing both BiTE and a test drug. This suggested that the tetracycline-based compound increased the granzyme B-expressing $CD8^+$T cells.

V. Example 4: Examination of Proliferative Capacity Enhancement of $CD8^+$T cells by Tetracycline-based Compound

According to the method described in section 1.3 above, using demethyl chlortetracycline (DMC: final concentration of 2.5 μM) as a test drug, an effect of the tetracycline-based compound on the proliferative capacity of $CD8^+$T cells in PBMCs that were individually collected from five healthy people were examined. As shown in Fig. 5, as compared to the PBMCs containing BiTE alone, the proliferative capacity of $CD8^+$T cells was high ($p = 0.007$) in the PBMCs containing BiTE and a test drug in combination. This suggested that the tetracycline-based compound enhanced the proliferative capacity of $CD8^+$ T cells having tumor-cytotoxic activity.

VI. Example 5: Examination of Effect of Induction Enhancement of CMV-specific Cytotoxic T Cells by Tetracycline-based Compound

According to the method described in section 1.4 above, an effect of a tetracycline-based compound in inducing CMV-specific cytotoxic T cells by stimulating PBMCs with CMV antigens was examined. As a test drug, demethyl chlortetracycline (DMC: final concentration of 2.5 μM) was used. The PBMCs were collected from one healthy person. The CMV-specific cytotoxic T cells were identified by FACS analysis. As shown in Fig. 6A, although the induction of CMV-specific cytotoxic T cells (fractionated in fraction P7 in Fig. 6A) was not observed 7 days after the stimulation of the CMV-specific cytotoxic T cells by inducing antigens, the induction of CMV-specific cytotoxic T cells was observed 14 days after the stimulation. Comparison between the group free of DMC (DMC-) and the group containing DMC (DMC+) indicated that the presence ratio of the CMV-specific cytotoxic T cells (CMV Tetramer + $CD8^+$T cells) was significantly high (Fig. 6B; $p<0.05$) in DMC+ as compared to DMC-. This suggested that the tetracycline-based compound enhanced the proliferative capacity of antigen-specific T cells.

VII. Example 6: Examination of Enhancement Effect of Tetracycline-based Compound on Tumor-Cytotoxic Activity of T Cells in Lung Cancer Tissue

According to the method described in section 1.5 above, T cells in tumor tissue of a lung cancer patient were collected; and by using such cells, tumor-cytotoxic activity was measured to examine the effect of the tetracycline-based compound. Specifically, T cells in tumor tissue of a lung cancer patient were collected according to the method described

in section 1.5 above; and the presence ratio of IFNγ-producing $CD8^+T$ cells was measured according to the method described in section 1.6 above, thus examining the effect of the tetracycline-based compound.

Demethyl chlortetracycline (DMC) was used as a test drug. n was 5 (independent patients) in the cytotoxic activity measurement, and n was 3 (independent patient) in the measurement of the presence ratio of IFNγ-producing $CD8^+T$ cells.

The combination of BiTE and a test drug enhanced the anti-tumor activity of T cells compared BiTE alone (Fig. 7A).

The combination of BiTE and a test drug increased the presence ratio of the IFNγ-producing $CD8^+T$ cells compared to BiTE alone (Fig. 7B and C). This suggested that the tetracycline-based compound increased IFNγ-producing $CD8^+T$ cells.

VIII. Example 7: Effect of Tetracycline-based Compound *in Vivo*

According to the method described in section 7 above, the tumor proliferation inhibition effect of the tetracycline-based compound *in vivo* was examined. As compared to the vehicle group, an increase in tumor size tended to be reduced in the test drug administration group (Fig. 8B).

The results indicated that the tetracycline-based compound had an effect of activating an anti-tumor action of T cells.

VIII. Example 8: Examination of Effect of Combined Use of Tetracycline-based Compound and Anti-PD-Ll Antibody *in Vivo*

Examination was conducted as to whether the tetracycline-based compound can enhance the anti-tumor effect of an immune checkpoint inhibitor *in vivo.*

1. Method

CT26WT cells were thawed six days before inoculation, and cultured in a DMEM medium containing 10% FBS and 1% penicillin/streptomycin. The CT26WT cells were treated with trypsin two days before the administration to tumor, followed by passage culture.

After trimming hair at the right dorsal side of 6-week-old BALB/c mice, the CT26WT cells were intradermally inoculated at $3 \times 10^5$ cells/50 μL/mouse (Day 0). The weight and tumor size of each mouse were measured on Day 6, and the mice were divided into 5 groups each containing 9 mice so that the groups had a uniform weight and tumor size. As an anti-PD-Ll antibody, InVivo MAb anti-mouse PD-L1 produced by Bio X Cell was used.

Details of groups are described below.

A group containing control IgG and water (control IgG + $H_2O$ group).
A group containing an anti-PD-Ll antibody and water (aPD-L1 + $H_2O$ group).
A group containing an anti-PD-Ll antibody and DMC (300 mg/kg) (aPD-L1 + DMC (300 mg/kg) group).
A group containing an anti-PD-Ll antibody and DMC (100 mg/kg) (aPD-L1 + DMC (100 mg/kg) group).
A group containing an anti-PD-Ll antibody and DMC (30 mg/kg) (aPD-L1 + DMC (30 mg/kg) group).

First, the administration of DMC was started on Day 7. The DMC was dissolved in saline, and then orally administered at 200 μL/mouse using an oral sonde. In the group free of DMC, distilled water was orally administered at 200 μL/mouse. The DMC was administered once daily from Day 7 to Day 12.

On Day 10, an anti-PD-Ll antibody or control IgG was intraperitoneally administered at 200 μg/200 μL/mouse.

On Day 10 and Day 13, the tumor size was measured. The tumor volume was calculated according to the following formula:

$$(\text{short diameter}^2 \times \text{long diameter})/2.$$

2. Results

Fig. 9 shows measurement results. In the control IgG+$H_2O$ group, the tumor volume, which was about 40 $mm^3$ at the start of the DMC administration, was increased to about 160 $mm^3$. In contrast, the tumor volume in the aPD-L1+$H_2O$ group was only about 90 $mm^3$. In all of the groups in which DMC had been previously administered, an increase in tumor volume was inhibited as compared to the aPD-L1+$H_2O$ group. In particular, the aPD-L1+DMC (30 mg/kg) group showed a great effect of inhibiting tumor volume increase.

This indicated that the administration of the tetracycline-based compound enhanced the anti-tumor effect of

the immune checkpoint inhibitor.

IX. Example 9: Examination of Whether Enhancement Effect of Tetracycline-based Compound on Anti-tumor Action of Anti-PD-Ll Antibody *in Vivo* Depends on $CD8^+T$ Cells

To examine whether the effect of enhancing the anti-tumor action of an immune checkpoint inhibitor *in vivo* demonstrated by the tetracycline-based compound depends on $CD8^+T$ cells, the effect of the tetracycline-based compound on the anti-tumor action of the immune checkpoint inhibitor was examined in the presence or absence of $CD8^+T$ cells.

1. Method

In the same manner as in Example 8, CT26WT cells were cultured, and subcutaneously inoculated to 6-week-old BALB/c mice (Day 0). On Day 6, the weight and tumor size of each mouse were measured, and the mice were divided into 6 groups each containing 9 mice so that the groups had a uniform weight and tumor size. A group containing control IgG and water (IgG+$H_2O$ group). A group containing an anti-PD-Ll antibody and water (aPD-L1+$H_2O$ group).
A group containing an anti-PD-Ll antibody, water, and an anti-CD8 antibody (aPD-L1+$H_2O$+CD8 depletion group).
A group containing an anti-PD-Ll antibody, DMC (30 mg/kg), and an anti-CD8 antibody (aPD-L1+DMC+CD8 depletion group).
A group containing control IgG and DMC (30 mg/kg) (IgG+DMC group).
A group containing an anti-PD-Ll antibody and DMC (30 mg/kg) (aPD-L1+DMC group).

Before the administration of DMC or water and an anti-PD-L1 antibody, the anti-CD8 antibody (InVivo MAb anti-mouse CD8$\alpha$, produced by BioXCell) was intraperitoneally administered at 400 $\mu$g/200 $\mu$L/mouse to the CD8 depletion group on Day 6. Control IgG was administered at 400 $\mu$g/200 $\mu$L/mouse to the other groups.

Next, administration of DMC was started on Day 7. The DMC was dissolved in saline, and then orally administered at 200 $\mu$L/mouse using an oral sonde. In the groups free of DMC, distilled water was orally administered at 200 $\mu$L/mouse. The DMC was administered once daily from Day 7 to Day 12.

Subsequently, on Day 10, an anti-PD-Ll antibody or control IgG was intraperitoneally administered at 200 $\mu$g/200 $\mu$L/mouse.

On Day 10 and Day 13, the tumor size was measured. The tumor volume was calculated in the same manner as in Example 8.

2. Results

Fig. 10 shows the results. In the IgG+$H_2O$ group, the tumor volume was about 150 $mm^3$ on Day 13 while the tumor volume in the aPD-L1+$H_2O$ group on Day 13 was about 120 $mm^3$. The increase in tumor volume was more inhibited in the IgG+DMC group as compared to the aPD-L1+$H_2O$ group, and the tumor volume on Day 13 was about 100 $mm^3$. In the aPD-L1+DMC group, the tumor volume on Day 13 was about 80 $mm^3$. In contrast, administration of an anti-CD8 antibody accelerated the rate of increase in tumor volume compared to the igG+$H_2O$ group, and in the aPD-L1+$H_2O$+CD8 depletion group and the aPD-L1+DMC+CD8 depletion group, the tumor volume was increased on Day 10 as compared to the IgG+$H_2O$ group. Moreover, the tumor volume was around 300 $mm^3$ on Day 13 in both the aPD-L1+$H_2O$+CD8 depletion group and the aPD-L1+DMC+CD8 depletion group, and the increase in tumor volume was accelerated even in the presence of the anti-PD-Ll antibody or in the presence of the anti-PD-Ll antibody and demethyl chlortetracycline. On Day 13, there was a significant decrease in tumor volume in the aPD-L1+DMC group as compared to the aPD-L1+$H_2O$ group, whereas there was no significant difference in tumor volume in the aPD-L1+DMC+CD8 depletion group and the aPD-L1+$H_2O$+CD8 depletion group.

The above indicated that the enhancement effect of the tetracycline-based compound on the anti-tumor action of the immune checkpoint inhibitor depends on CD8+T cells.

X. Example 10: Examination of Effect of Tetracycline-based Compound on Cancer Antigen-specific $CD8^+T$ cells *in Vivo*

It was examined whether the cancer antigen-specific $CD8^+T$ cells are increased *in vivo* by the anti-tumor action enhancement effect of the tetracycline-based compound on the immune checkpoint inhibitor.

1. Method

On Day 13, blood was collected from the eye fundus of each mouse in the IgG+$H_2O$ group, the aPD-L1+$H_2O$ group, and the aPD-L1+DMC group in Example 9. 1 ml of pharmlyse was added to a tube containing the collected blood

to lyse red blood cells, and after centrifugation, the supernatant was removed. An HBSS medium containing 2% FBS and 10mM HEPES ("HBSS+2% FBS+10mM HEPES") was added to the tube containing the sediment to suspend the sediment, and then centrifugation was performed again to remove the supernatant. Gp 70 tetramer (T-Select H-2Ld MuLV gp70 Tetramer-SPSYVYHQF-PE, produced by MBL) was added to the tube containing the sediment, followed by reaction at 4°C for 30 minutes. As a control, β-galactosidase tetramer (T-Select H-2Ld β-galactosidase Tetramer-TPHPARIGL-PE, produced by MBL) was added to the tube containing the sediment, followed by reaction at 4°C for 30 minutes. HBSS+2%FBS+10mM HEPES was added to the tube obtained after the completion of reaction, and mixed. The tube was centrifuged again to remove the supernatant. After the anti-CD8 antibody was added to the tube, and staining was performed, the cells were washed and subjected to a FACS measurement. The number of cells positive for gp70 tetramer and CD8 were counted.

2. Results

The counting results are shown in Fig. 11. Compared to the IgG+$H_2O$ group, the presence ratio of cancer antigen-specific $CD8^+$ cells was increased in the aPD-L1+DMC group. This revealed that the tetracycline-based compound increased the cancer antigen-specific $CD8^+$T cells when it enhanced the anti-tumor action of the immune checkpoint inhibitor.

## Claims

**1.** A tumor immunomodulator comprising at least one member selected from the group consisting of a tetracycline-based compound represented by the following formula (I) and its pharmaceutically acceptable salt:

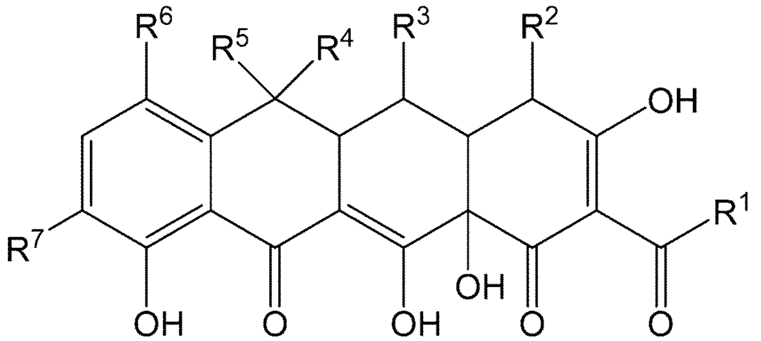

(I)

wherein
$R^1$ is a $C_{1-3}$ lower alkyl group or a group represented by the following formula (II):

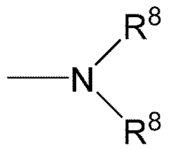

(II)

wherein $R^8$ is a hydrogen atom or a $C_{1-3}$ lower alkyl group; $R^2$ is a group represented by the following formula (III):

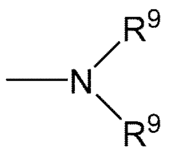

(III)

wherein $R^9$ is a $C_{1-3}$ lower alkyl group or a hydrogen atom;
$R^3$ is a hydrogen atom, a hydroxyl group, or a $C_{1-3}$ lower alkyl group;
$R^4$ and $R^5$ are the same or different and each is a hydrogen atom, a hydroxyl group, or a $C_{1-3}$ lower alkyl group, or $R^4$ and $R^5$ taken together are a methylene group;
$R^6$ is a hydrogen atom, halogen, or a group represented by the following formula (IV):

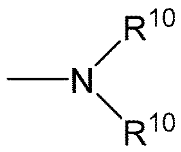 (IV)

wherein $R^{10}$ is a hydrogen atom or a $C_{1-3}$ lower alkyl group; and
$R^7$ is a hydrogen atom, a $C_{1-3}$ lower alkyl group, $-NH-CO-CH_2-NH-C\ (CH_3)_3$, or $-CH_2-NH-CH_2-C(CH_3)_3$.

**2.** The tumor immunomodulator according to claim 1, wherein the $C_{1-3}$ lower alkyl group is a methyl group.

**3.** The tumor immunomodulator according to claim 1, wherein the compound represented by formula (I) is at least one member selected from the group consisting of demethyl chlortetracycline, meclocycline, tetracycline, chlortetracycline, doxycycline, minocycline, and oxytetracycline.

**4.** The tumor immunomodulator according to any one of claims 1 to 3, for use in combination with a tumor immunotherapy agent.

**5.** The tumor immunomodulator according to claim 4, wherein the tumor immunotherapy agent is at least one member selected from the group consisting of immune checkpoint inhibitors, CAR-T cell drugs, bispecific molecule drugs, and cancer vaccines.

**6.** A composition for tumor treatment comprising the tumor immunomodulator according to any one of claims 1 to 3 and a tumor immunotherapy agent.

**7.** The composition for tumor treatment according to claim 6, wherein the tumor immunotherapy agent is at least one member selected from the group consisting of immune checkpoint inhibitors, CAR-T cell drugs, bispecific molecule drugs, and cancer vaccines.

Fig.1

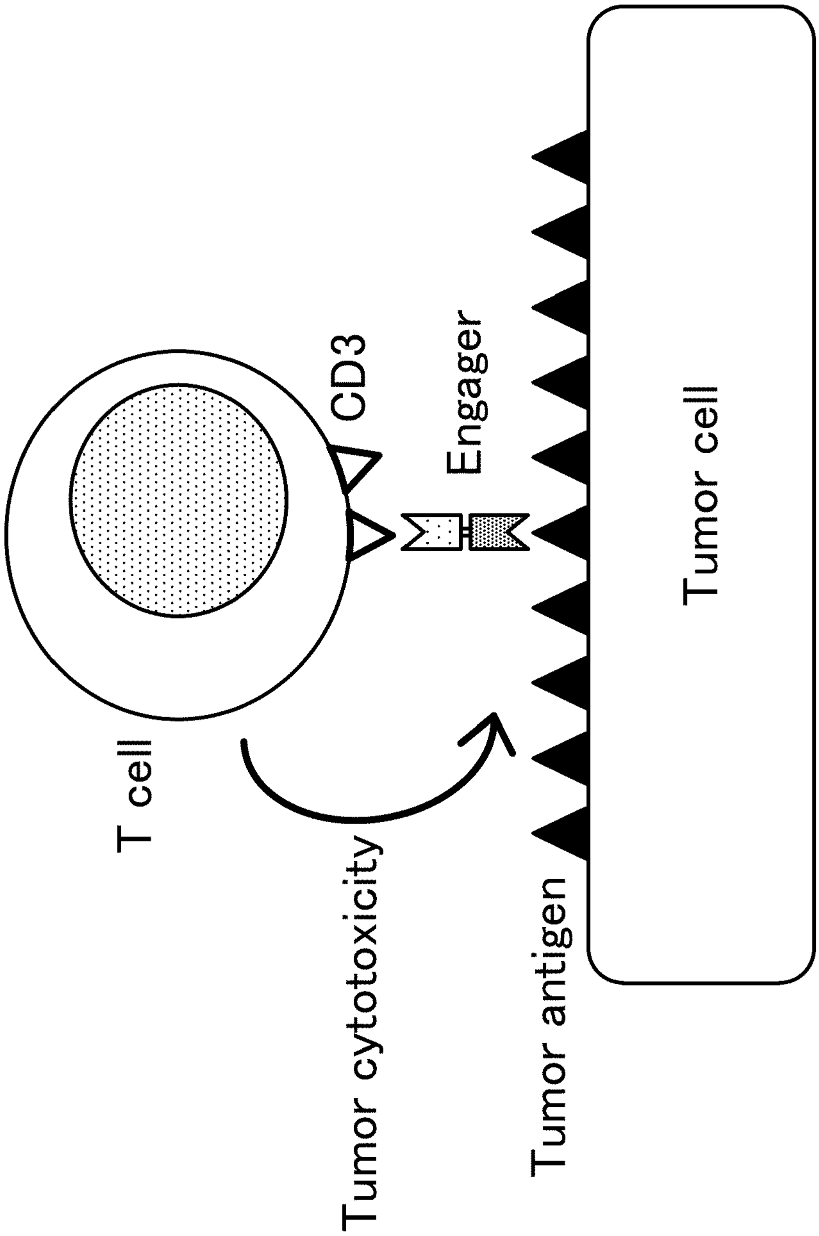
T cell
CD3
Engager
Tumor cytotoxicity
Tumor antigen
Tumor cell

Fig.2

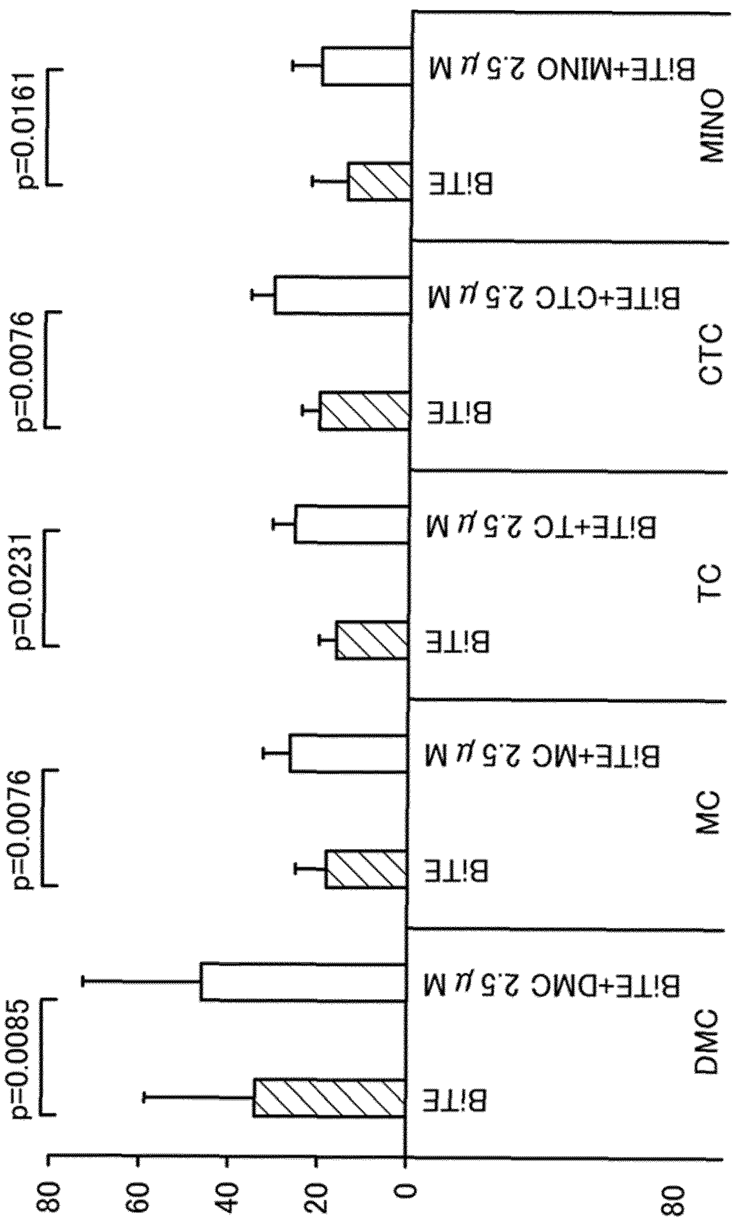

Tumor-cytotoxic Activity (%)
0
20
40
60
80
80
p=0.0085
p=0.0076
p=0.0231
p=0.0076
p=0.0161
BiTE
BiTE+DMC 2.5 μM
DMC
BiTE
BiTE+MC 2.5 μM
MC
BiTE
BiTE+TC 2.5 μM
TC
BiTE
BiTE+CTC 2.5 μM
CTC
BiTE
BiTE+MINO 2.5 μM
MINO

Fig. 3

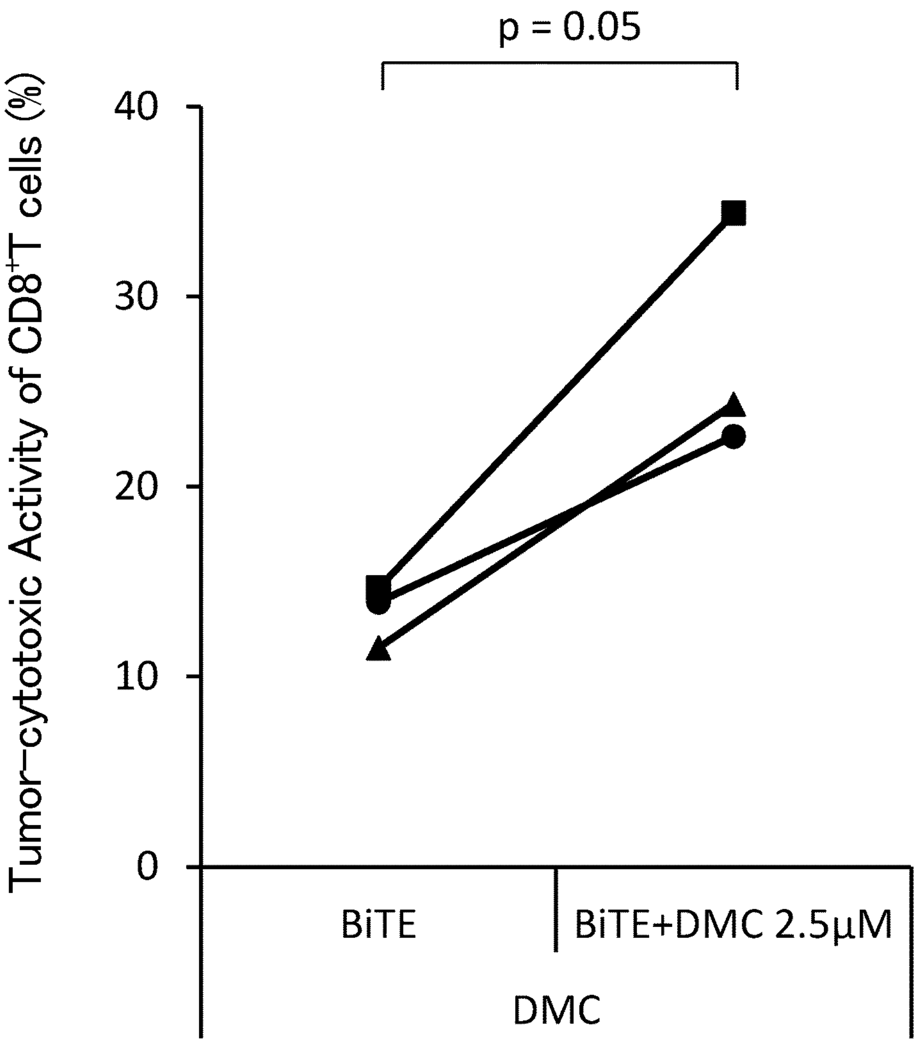
p = 0.05
Tumor-cytotoxic Activity of CD8+T cells (%)
40
30
20
10
0
BiTE
BiTE+DMC 2.5μM
DMC

Fig. 4

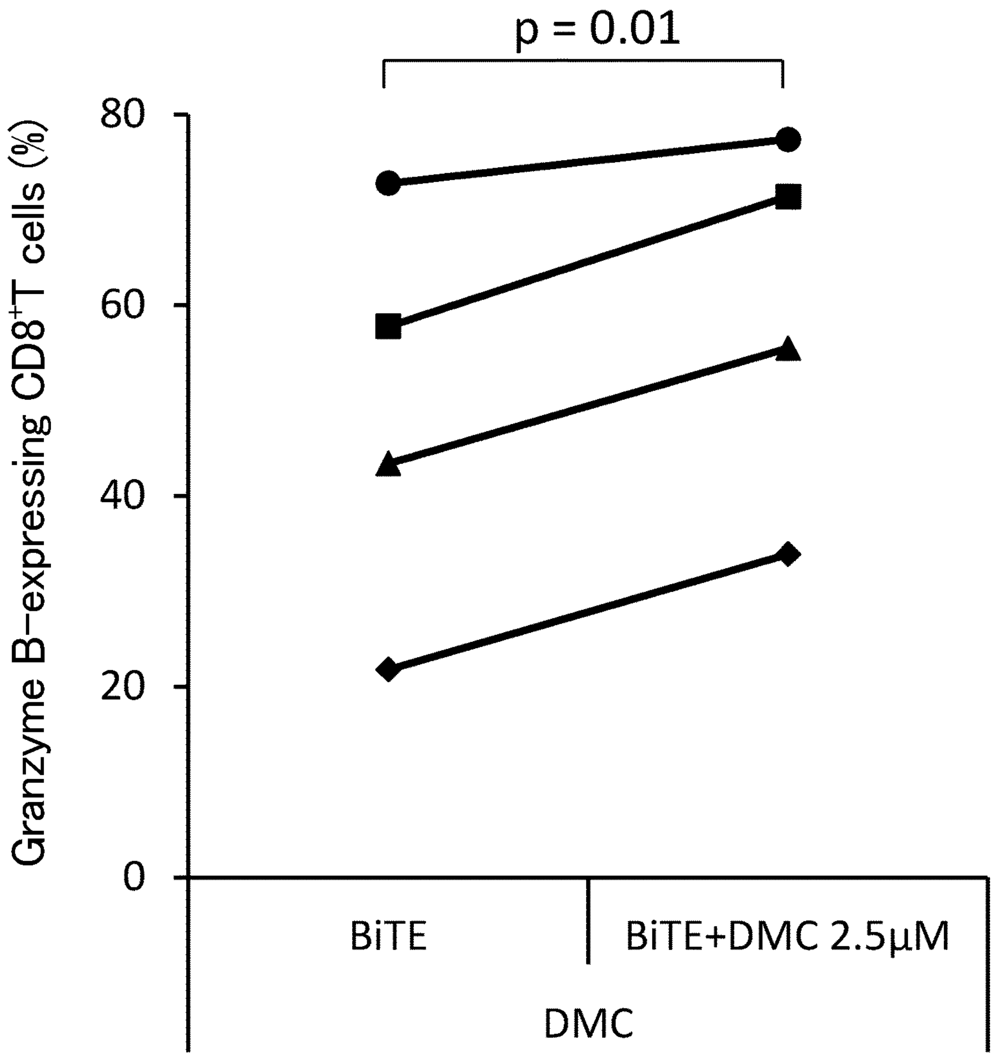
p = 0.01
Granzyme B-expressing CD8+T cells (%)
80
60
40
20
0
BiTE
BiTE+DMC 2.5μM
DMC

Fig. 5

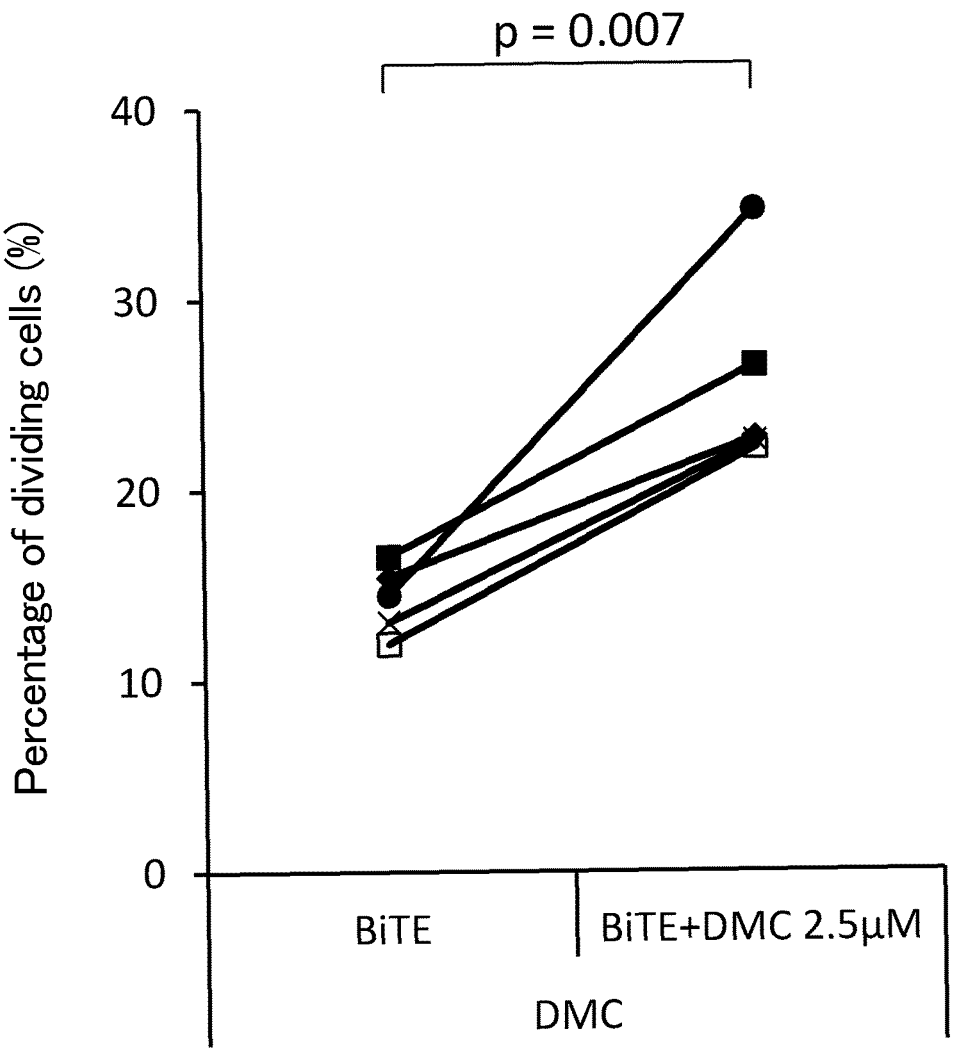
p = 0.007
40
30
20
10
0
Percentage of dividing cells (%)
BiTE
BiTE+DMC 2.5μM
DMC

Fig.6

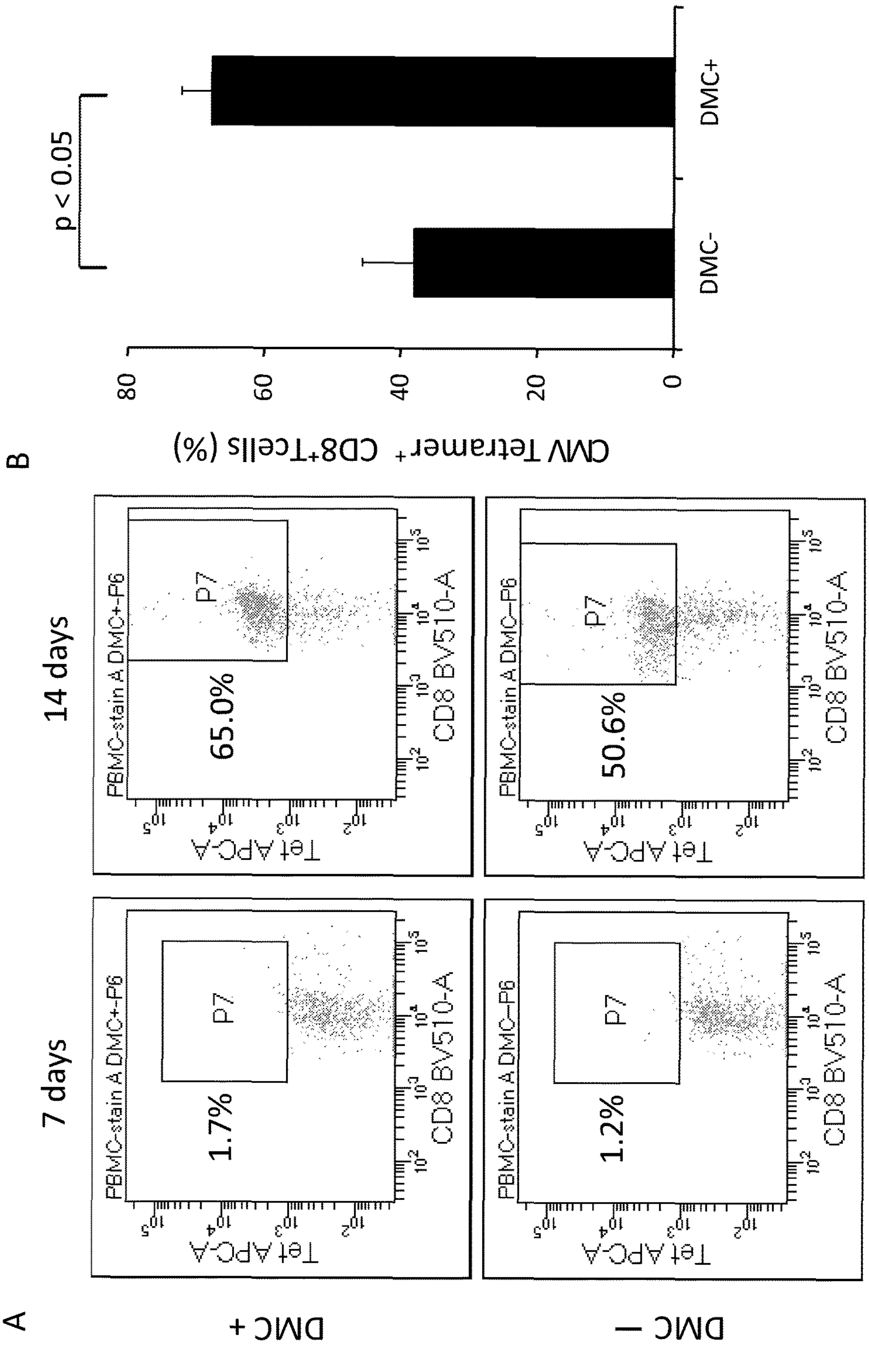
A
7 days
14 days
DMC +
DMC −
PBMC-stain A DMC+-P6
PBMC-stain A DMC−-P6
P7
1.7%
1.2%
65.0%
50.6%
Tet APC-A
CD8 BV510-A
B
CMV Tetramer+ CD8+Tcells (%)
0
20
40
60
80
DMC-
DMC+
p < 0.05

Fig.7

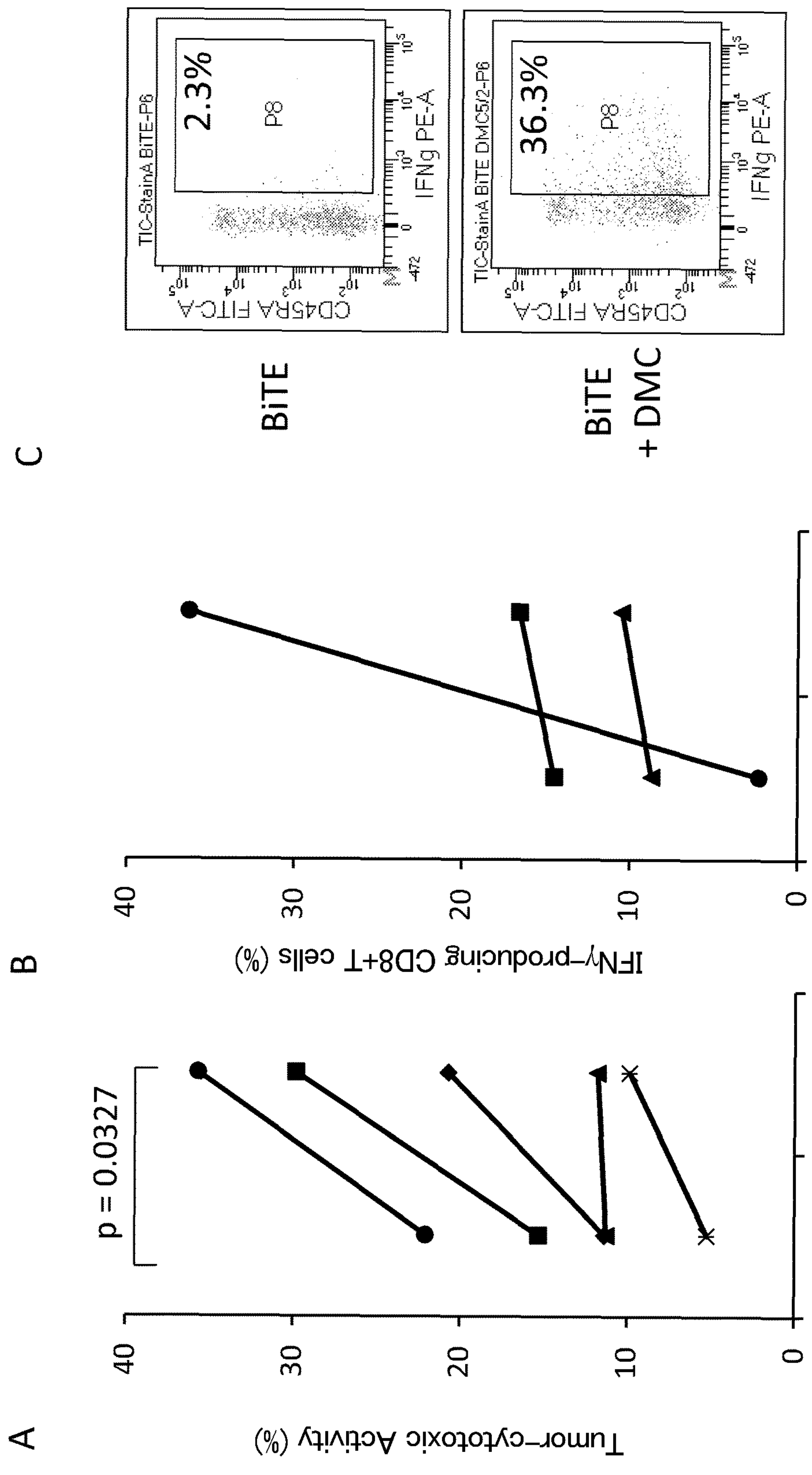
A
Tumor−cytotoxic Activity (%)
40
30
20
10
0
p = 0.0327
BiTE
BiTE+DMC
2.5μM
B
IFNγ−producing CD8+T cells (%)
40
30
20
10
0
BiTE
BiTE+DMC
2.5μM
C
BiTE
BiTE
+ DMC
2.3%
P8
36.3%
CD45RA FITC-A
IFNg PE-A

Fig.8

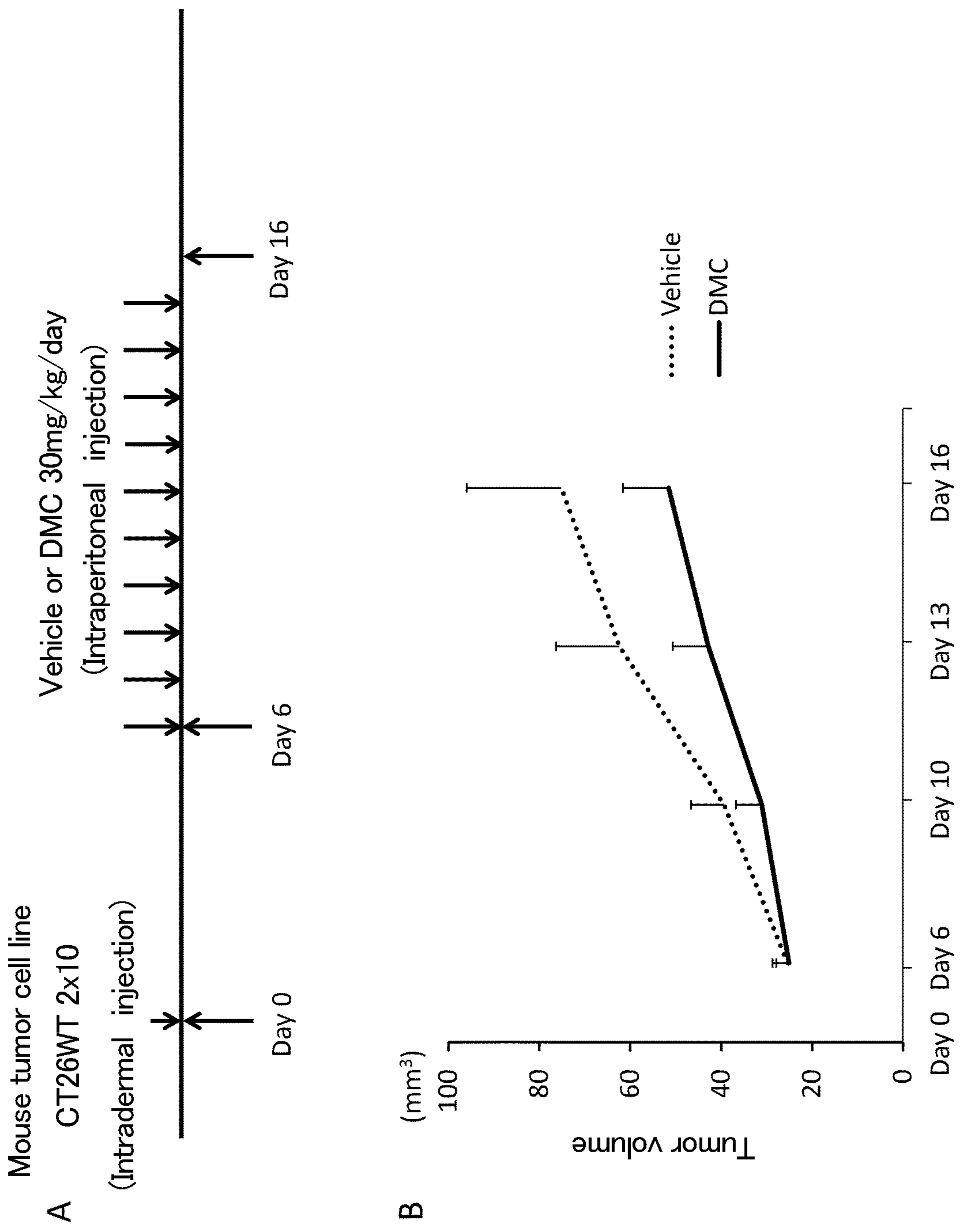
A
Mouse tumor cell line
CT26WT 2x10
(Intradermal injection)
Day 0
Day 6
Day 16
Vehicle or DMC 30mg/kg/day
(Intraperitoneal injection)
B
Tumor volume
(mm3)
100
80
60
40
20
0
Day 0
Day 6
Day 10
Day 13
Day 16
Vehicle
DMC

Fig.9

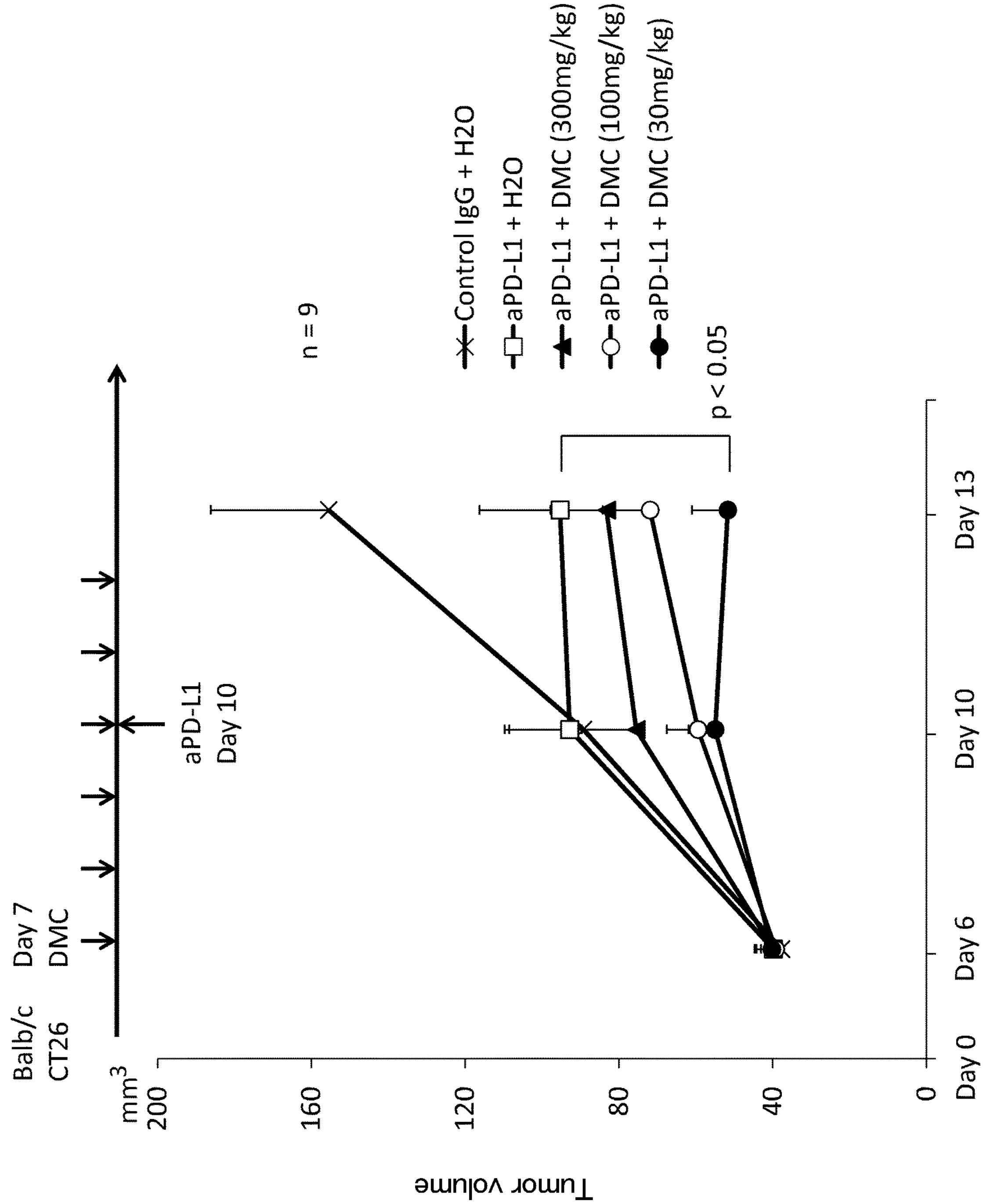
Balb/c
CT26
Day 7
DMC
aPD-L1
Day 10
n = 9
Control IgG + H2O
aPD-L1 + H2O
aPD-L1 + DMC (300mg/kg)
aPD-L1 + DMC (100mg/kg)
aPD-L1 + DMC (30mg/kg)
p < 0.05
Tumor volume
mm3
200
160
120
80
40
0
Day 0
Day 6
Day 10
Day 13

Fig.10

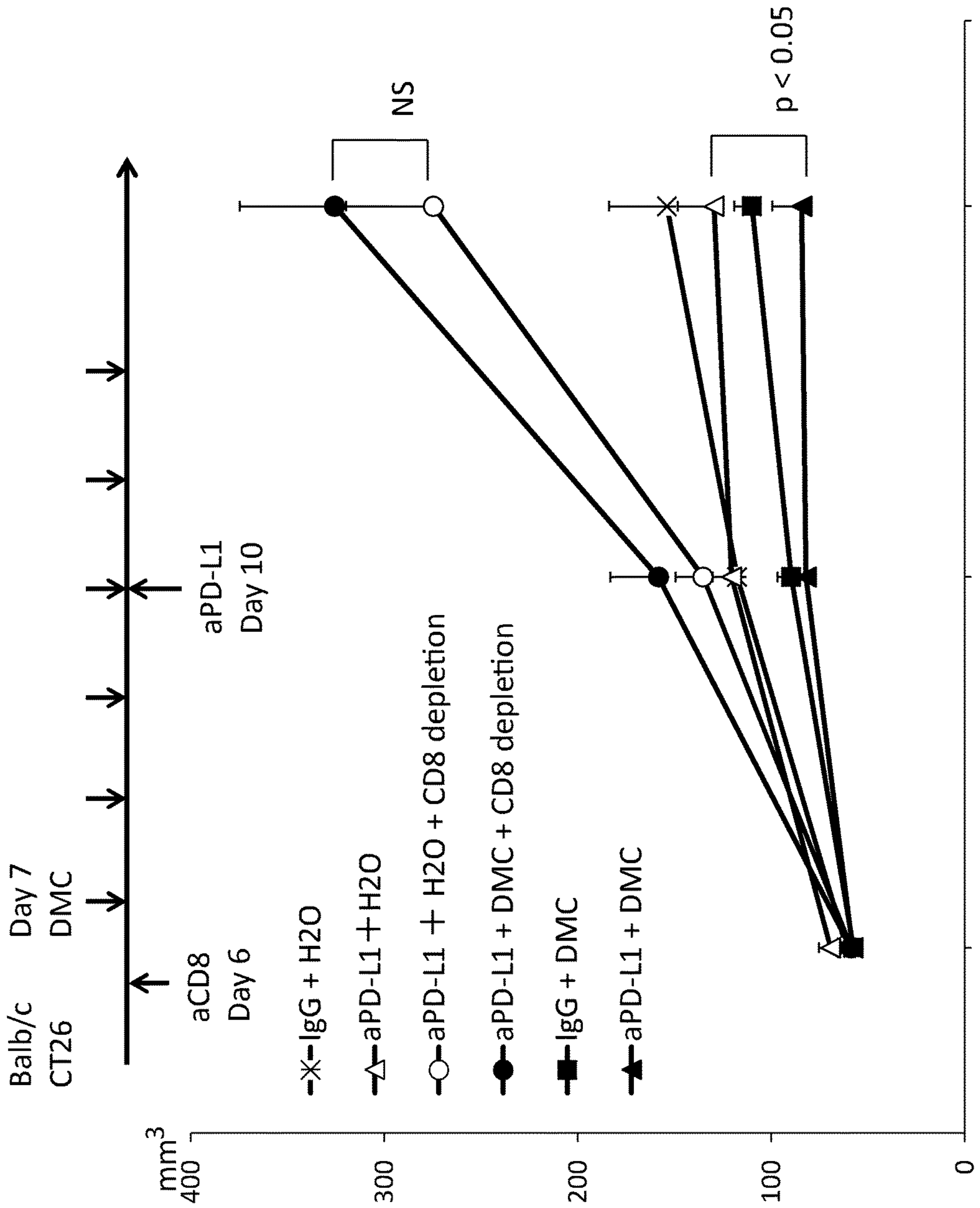

Balb/c
CT26
aCD8
Day 6
Day 7
DMC
aPD-L1
Day 10
IgG + H2O
aPD-L1 + H2O
aPD-L1 + H2O + CD8 depletion
aPD-L1 + DMC + CD8 depletion
IgG + DMC
aPD-L1 + DMC
NS
p < 0.05
Tumor volume
mm3
400
300
200
100
0
Day 0
Day 6
Day 10
Day 13

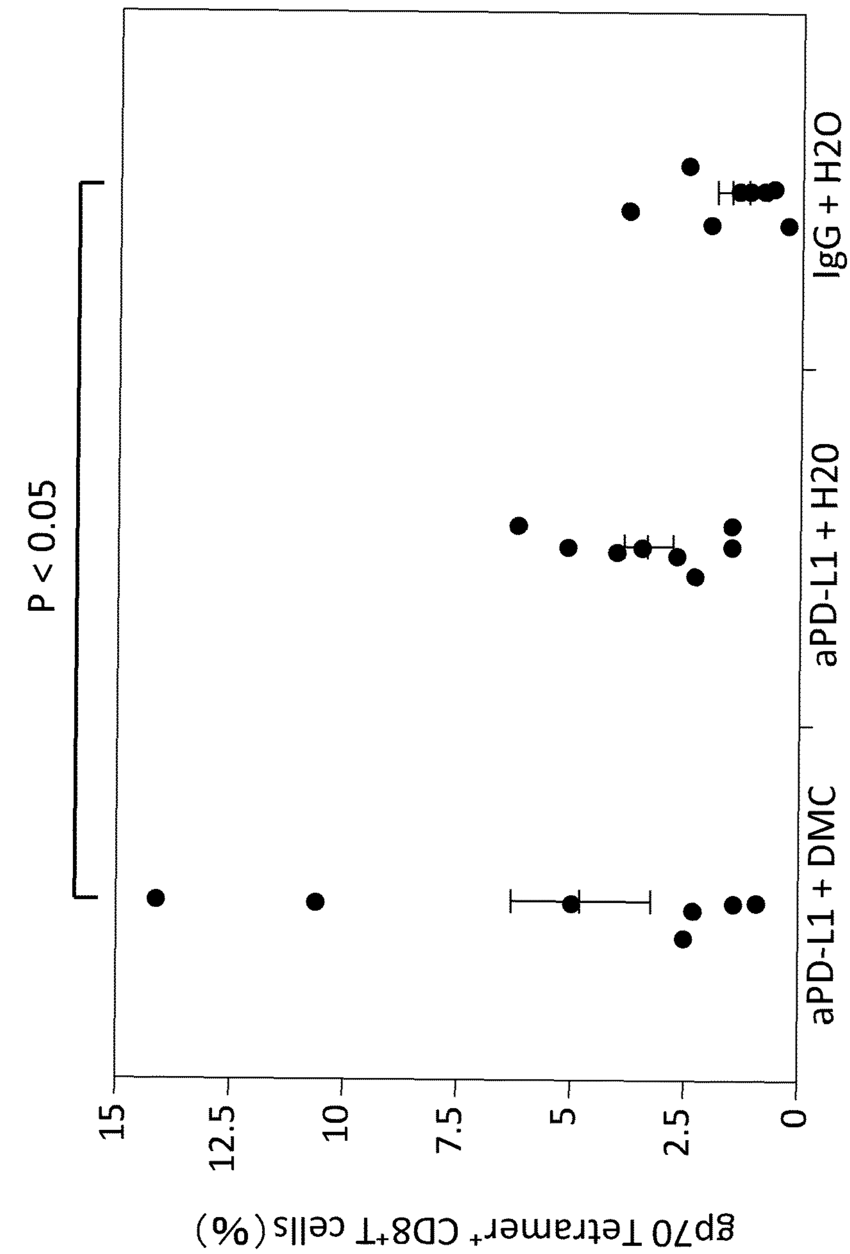
P < 0.05
aPD-L1 + DMC
aPD-L1 + H2O
IgG + H2O
gp70 Tetramer+ CD8+T cells (%)
0
2.5
5
7.5
10
12.5
15

Fig.11

**INTERNATIONAL SEARCH REPORT**

International application No. PCT/JP2019/010236

A. CLASSIFICATION OF SUBJECT MATTER
Int.Cl. A61K31/65(2006.01)i, A61P35/00(2006.01)i, A61P37/04(2006.01)i, A61P43/00(2006.01)i

According to International Patent Classification (IPC) or to both national classification and IPC

B. FIELDS SEARCHED

Minimum documentation searched (classification system followed by classification symbols)
Int.Cl. A61K31/65, A61P35/00, A61P37/04, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | TANG, H. et al., Potential for enhanced therapeutic activity of biological cancer therapies with doxycycline combination., Gene Ther., July 2013, vol. 20, no. 7, pp. 770-778, abstract, p. 775, left column, ll. 1-6, fig. 4 | 1-4, 6<br>1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11.04.2019 | 23.04.2019 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No. PCT/JP2019/010236

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | 岩崎博道，テトラサイクリンにより誘導されるヒト白血病細胞のアポトーシス，臨床薬理，31 March 2000, vol. 31, no. 2, ISSN 0388-1601, pp. 337, 338, [Introduction], [Table], p. 338, left column, [Consideration], ll. 16-19, right column, ll. 5-12, (IWASAKI, Hiromichi, Japanese Journal of Clinical Pharmacology and Therapeutics), non-official translation (Apoptosis of induced human leukemia cells by tetracycline) | 1-3<br>1-7 |
| X<br>Y | ACHARYA, M. R. et al., Chemically modified tetracyclines as inhibitors of matrix metalloproteinases, Drug Resist Updat., January 2004, vol. 7, no. 3, pp. 195-208, p. 198, left column, [3], ll. 12-15, right column, [4], ll. 17-19, p. 201, left column, ll. 3-7, fig. 2 | 1-3<br>1-7 |
| Y | JP 2006-340714 A (ONO PHARMACEUTICAL CO., LTD.) 21 December 2006, claims, paragraph [0003] & US 2009/0217401 A1, claims, paragraph [0003] & WO 2006/121168 A1 & EP 1896582 A1 & CN 101213297 A & KR 10-2013-0032908 A | 4-7 |
| Y | JP 2002-512020 A (MICROMET AG) 23 April 2002, claims, paragraphs [0008], [0009] & US 2006/0193852 A1, claims, paragraphs [0044]-[0047] & WO 1999/054440 A1 & EP 1071752 A1 & CN 1299410 A | 4-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Anticancer Drugs,* September 2013, vol. 24 (8), 799-809
- *Curr Med Chem,* February 2001, vol. 8 (3), 271-279